# EUROPÄISCHE PATENTSCHRIFT

(11) **EP 1 326 866 B1**
(45) Veröffentlichungstag und Bekanntmachung des Hinweises auf die Patenterteilung: **15.06.2005**
(21) Anmeldenummer: 01978402.4
(22) Anmeldetag: 10.10.2001
(51) Int. Cl.: C07D 487/04, A61P 29/00

(54) **SUBSTITUIERTE 3,4-DIHYDRO-PYRIDO[1,2-A]PYRIMIDINE**
SUBSTITUTED 3.4-DIHYDRO-PYRIDO[1,2-A]PYRIMIDINES
3,4-DIHYDRO-PYRIDO[1,2-A]PYRIMIDINES SUBSTITUEES

(30) Priorität: 13.10.2000 DE 10050662
(43) Veröffentlichungstag der Anmeldung: 16.07.2003
(73) Patentinhaber: Grünenthal GmbH, 52078 Aachen (DE)
(72) Erfinder: GERLACH, Matthias, 63636 Brachttal (DE); MAUL, Corinna, 52066 Aachen (DE); JAGUSCH, Utz-Peter, 52066 Aachen (DE)
(86) Internationale Anmeldenummer: PCT/EP2001/011700
(87) Internationale Veröffentlichungsnummer: WO 2002/030933

(56) Entgegenhaltungen:
- M. A. ABDEL-RAHMAN: "Invers electron demand diels-alder reactioins of 2-(arylmethyleneamin)-pyridines with enamines and styrenes. Synthesis of pyramidine derivatives" REVUE ROUMAINE DE CHIMIE, Bd. 40, 1995, Seiten 535-540, XP002188368
- L.S. POVAROV: "reaction of pyridylimines of aromatic aldehydes with unsaturated ethers" CHEM. HETEROCYCL. COMP., Bd. 15, 1979, Seite 1369 XP002188369
- ALAN. R. KATHRITZKY, GUOFANG QIU, BAOZHEN YANG: "A novel facile method for thge synthesis of 3,4-dihydro-2H-pyrido[1,2-a]pyrimidinium salts" SYNTHESIS, Bd. 5 , 1998, Seiten 704-706, XP002188370

## Beschreibung

Die vorliegende Anmeldung betrifft substituierte 3,4-Dihydro-pyrido[1,2-a]pyrimidine, Verfahren zu ihrer Herstellung, Arzneimittel, die diese Verbindungen enthalten, die Verwendung dieser Verbindungen zur Herstellung von Arzneimitteln zur Behandlung von Schmerz, Harninkontinenz, Juckreiz, Tinnitus aurium und/oder Diarrhoe sowie diese Verbindungen enthaltende pharmazeutische Zusammensetzungen.

Die Behandlung chronischer und nicht chronischer Schmerzzustände hat in der Medizin eine große Bedeutung. Es besteht ein weltweiter Bedarf an gut wirksamen Therapien für eine patientengerechte und zielorientierte Behandlung chronischer und nicht chronischer Schmerzzustände, wobei hierunter die erfolgreiche und zufriedenstellende Schmerzbehandlung für den Patienten zu verstehen ist.

Klassische Opioide wie Morphin sind bei der Therapie starker bis stärkster Schmerzen gut wirksam. Ihr Einsatz wird jedoch durch die bekannten Nebenwirkungen, wie z.B. Atemdepression, Erbrechen, Sedierung, Obstipation und Toleranzentwicklung, limitiert. Außerdem sind sie bei neuropathischen oder inzidentiellen Schmerzen, unter denen insbesondere Tumorpatienten leiden, weniger wirksam.

Der vorliegenden Erfindung liegt die Aufgabe zugrunde, analgetisch wirksame Verbindungen zur Verfügung zu stellen, die sich zur Schmerztherapie - insbesondere auch zur Therapie chronischer und neuropathischer Schmerzen - eignen. Darüber hinaus sollten diese Substanzen möglichst keine der Nebenwirkungen, die üblicherweise bei der Anwendung von Opioiden mit µ-Rezeptoraffinität wie Morphin auftreten, wie z.B: Übelkeit, Erbrechen, Abhängigkeit, Atemdepression oder Obstipation, hervorrufen.

Diese Aufgabe wird durch die Verbindungen der allgemeinen Struktur I gelöst, die analgetisch wirksam sind. Bei den erfindungsgemäßen Verbindungen handelt es sich um substituierte 3,4-Dihydro-pyrido[1,2-a]pyrimidine der allgemeinen Struktur I 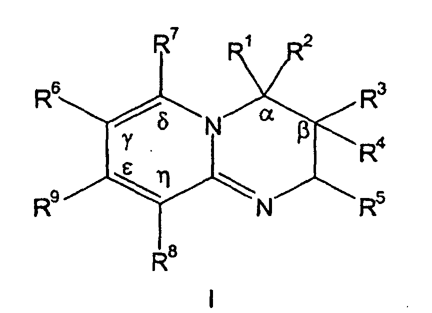 worin
- R¹ und R²: unabhängig voneinander H oder Phenyl oder Naphthyl, wobei Phenyl oder Naphthyl unsubstituiert oder ein- oder mehrfach mit OH oder OCH₃ substituiert sind, bedeuten, wobei mindestens ein Rest von R¹ und R² H ist,
- R³ und R⁴: unabhängig voneinander H oder C₁₋₁₂-Alkyl, wobei C₁₋₁₂-Alkyl geradkettig oder verzweigt ist, bedeuten, wobei mindestens ein Rest von R³ und R⁴ H ist,
- R⁵: C₃₋₈-Cycloalkyl, wobei Cycloalkyl gesättigt ist und unsubstituiert oder mit CO₂Ethyl substituiert ist, Furanyl substituiert mit NO₂, C(=O)R¹¹, CO₂H oder CO₂R¹² bedeutet,
- R⁶ und R⁸: beide Cl oder Br bedeuten,
- R⁷: H oder C₁₋₁₂-Alkyl, wobei Alkyl geradkettig oder verzweigt ist, bedeutet,
- R⁹: H bedeutet,
- R¹¹: Phenyl ist,
- R¹²: C₁₋₈-Alkyl, wobei Alkyl geradkettig oder verzweigt ist, bedeutet,
sowie ihre pharmazeutisch annehmbaren Salze.

Die Ausdrücke "C₁₋₆-Alkyl", "C₁₋₈-Alkyl" und "C₁₋₁₂-Alkyl" umfassen im Sinne dieser Erfindung acyclische gesättigte Kohlenwasserstoffreste, die verzweigt- oder geradkettig sein können, mit 1 bis 6 bzw. 1 bis 8 bzw. 1 bis 12 Kohlenstoffatomen, d.h. C₁₋₆-Alkanyle, bzw. C₁₋₈-Alkanyle, bzw. C₁₋₁₂-Alkanyle. Vorteilhaft ist Alkyl aus der Gruppe ausgewählt, die Methyl, Ethyl, n-Propyl, 2-Propyl, n-Butyl, iso-Butyl, sec.-Butyl, tert.-Butyl, n-Pentyl, isoPentyl, neo-Pentyl, n-Hexyl, 2-Hexyl, n-Octyl, n-Decyl und n-Dodecyl umfaßt.

Der Ausdruck "C₃₋₈-Cycloalkyl" bedeutet für die Zwecke dieser Erfindung cyclische Kohlenwasserstoffe mit 3 bis 8 Kohlenstoffatomen, die gesättigt, unsubstituiert oder substituiert sein können. Vorteilhaft ist C₃₋₈-Cycloalkyl aus der Gruppe ausgewählt, die Cyclopropyl, Cyclobutyl, Cyclopentyl, Cyclohexyl, Cycloheptyl, Cyclooctyl, Cyclopentenyl, Cyclohexenyl, Cycloheptenyl und Cyclooctenyl enthält. Für die Zwecke der vorliegenden Erfindung besonders bevorzugt sind Cyclopropyl, Cyclopropyl-2-carbonsäure, Cyclopropyl-2-carbonsäureethylester und Cyclohexyl.

Pharmazeutisch annehmbare Salze im Sinne dieser Erfindung sind solche Salze der erfindungsgemäßen Verbindungen gemäß der allgemeinen Struktur I, die bei pharmazeutischer Verwendung physiologisch - insbesondere bei Anwendung am Säugetier und/oder Menschen - verträglich sind. Solche pharmazeutisch annehmbaren Salze können beispielsweise mit anorganischen oder organischen Säuren gebildet werden.

Vorzugsweise werden die pharmazeutisch annehmbaren Salze der erfindungsgemäßen Verbindungen gemäß der allgemeinen Struktur I mit Salzsäure, Bromwasserstoffsäure, Schwefelsäure, Phosphorsäure, Methansulfonsäure, p-Toluolsulfonsäure, Kohlensäure, Ameisensäure, Essigsäure, Oxalsäure, Bernsteinsäure, Weinsäure, Mandelsäure, Fumarsäure, Milchsäure, Citronensäure, Glutaminsäure oder Asparaginsäure gebildet. Bei den gebildeten Salzen handelt es sich u.a. um Hydrochloride, Hydrobromide, Phosphate, Carbonate, Hydrogencarbonate, Formiate, Acetate, Oxalate, Succinate, Tartrate, Fumarate, Citrate und Glutaminate. Ebenfalls bevorzugt sind die Hydrate der erfindungsgemäßen Verbindungen, die z.B. durch Kristallisation aus wäßriger Lösung erhalten werden können.

Alle erfindungsgemäßen Verbindungen enthalten mindestens ein Asymmetriezentrum, nämlich das mit R⁵ substituierte Kohlenstoffatom der Struktur I. Daher können die erfindungsgemäßen Verbindungen der allgemeinen Struktur I in Form ihrer Racemate, in Form der reinen Enantiomeren und/oder Diastereomeren oder in Form von Mischungen dieser Enantiomeren bzw. Diastereomeren vorliegen, und zwar sowohl in Substanz als auch als pharmazeutisch annehmbare Salze dieser Verbindungen. Die Mischungen können in jedem beliebigen Mischungsverhältnis der Stereoisomeren vorliegen. Bevorzugt liegen die Verbindungen der allgemeinen Struktur I als enantiomerenreine Verbindungen vor.

Bevorzugte Verbindungen der vorliegenden Erfindung sind solche 3,4-Dihydro-pyrido[1,2-a]pyrimidine der allgemeinen Struktur I, in denen einer der Reste R¹ und R² Naphthyl bedeutet oder Phenyl ein- oder mehrfach substituiert mit OH oder OCH₃ bedeutet, einer der Reste R³ und R⁴ H oder C₁₋₆-Alkyl bedeutet, und die beiden anderen Reste von R¹, R², R³ und R⁴ H bedeuten, R⁵ C₃₋₆-Cycloalkyl, Furanyl substituiert mit NO₂, C(=O)R¹¹ oder CO₂R¹² bedeutet, R⁶ und R⁸ beide Cl oder Br bedeuten, R⁷ H oder C₁₋₆-Alkyl bedeutet, R⁹ H bedeutet, R¹¹ Phenyl und R¹² C₁₋₆-Alkyl bedeuten, sowie ihre pharmazeutisch annehmbaren Salze.

Insbesondere bevorzugt sind Verbindungen der allgemeinen Struktur I, in denen einer der Reste R¹ und R² 24-Hydroxyphenyl, 4-Methoxyphenyl, 4-Hydroxy-3-methoxyphenyl oder 2-Naphthyl bedeutet, einer der Reste R³ und R⁴ H oder Methyl bedeutet, und die beiden anderen Reste von R¹, R², R³ und R⁴ H bedeuten, R⁵ Cyclopropyl, 2-(C(=O)O-Ethyl)-cyclopropyl, Cyclohexyl, 5-Nitro-furan-2-yl, C(=O)Phenyl, oder CO₂Ethyl bedeutet, R⁶ und R⁸ beide Cl oder Br bedeuten, R⁷ H oder Methyl bedeutet und R⁹ H bedeutet, sowie ihre pharmazeutisch annehmbaren Salze.

Die am meisten bevorzugten erfindungsgemäßen Verbindungen sind jene, die ausgewählt sind aus:
[7,9-Dichlor-4-(4-hydroxy-3-methoxy-phenyl)-3-methyl-3,4-dihydro-2H-pyrido[1,2-a]pyrimidin-2-yl]-phenyl-methanon,
[7,9-Dibrom-4-(4-hydroxy-phenyl)-3,6-dimethyl-3,4-dihydro-2H-pyrido[1,2-a]pyrimidin-2-yl]-phenyl-methanon,
7,9-Dichlor-2-cyclohexyl-4-(4-methoxy-phenyl)-3,4-dihydro-2H-pyrido[1,2-a]pyrimidin,
7,9-Dibrom-2-cyclohexyl-4-(4-methoxy-phenyl)-3,4-dihydro-2H-pyrido[1,2-a]pyrimidin,
7,9-Dibrom-4-(3,4-dimethoxy-phenyl)-6-methyl-2-(5-nitro-furan-2-yl)-3,4-dihydro-2H-pyrido[1,2-a]pyrimidin,
2-[7,9-Dibrom-4-(4-methoxy-phenyl)-6-methyl-3,4-dihydro-2H-pyrido[1,2-a]pyrimidin-2-yl]-cyclopropancarbonsäureethylester,

Die Erfindung betrifft auch ein Verfahren zur Herstellung der erfindungsgemäßen Verbindungen der Struktur I 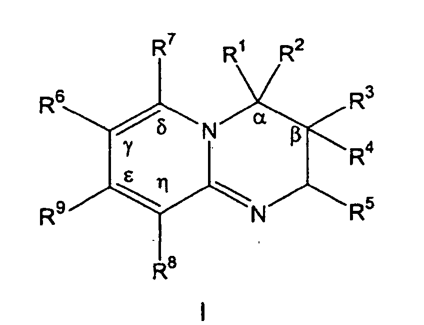 worin
- R¹ und R²: unabhängig voneinander H oder Phenyl oder Naphthyl, wobei Phenyl oder Naphthyl unsubstituiert oder ein- oder mehrfach mit OH oder OCH₃ substituiert sind, bedeuten, wobei mindestens ein Rest von R¹ und R² H ist,
- R³ und R⁴: unabhängig voneinander H oder C₁₋₁₂-Alkyl, wobei C₁₋₁₂-Alkyl geradkettig oder verzweigt ist, bedeuten, wobei mindestens ein Rest von R³ und R⁴ H ist,
- R⁵: C₃₋₈-Cycloalkyl, wobei Cycloalkyl gesättigt ist und unsubstituiert oder mit CO₂Ethyl substituiert ist, Furanyl substituiert mit NO₂, C(=O)R¹¹ oder CO₂R¹² bedeutet,-
- R⁶ und R⁸: beide Cl oder Br,
- R⁷: H oder C₁₋₁₂-Alkyl, wobei Alkyl geradkettig oder verzweigt ist, bedeutet,
- R⁹: H bedeutet,
- R¹¹: Phenyl ist,
- R¹²: C₁₋₈-Alkyl, wobei Alkyl geradkettig oder verzweigt ist, bedeutet,
sowie ihre pharmazeutisch annehmbaren Salze,
wobei das Verfahren dadurch gekennzeichnet ist, daß ein Heteroarylamin der allgemeinen Struktur II 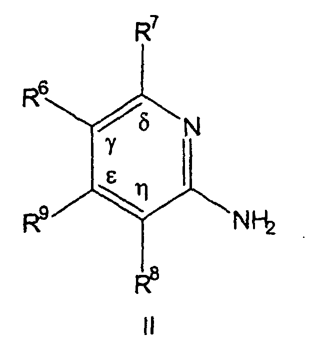 worin R⁶ , R⁷ , R8 und R9 wie oben definiert sind,
mit einem Aldehyd der allgemeinen Struktur III 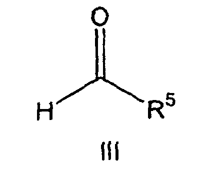 worin R⁵ wie oben definiert ist,
und einem Olefin der allgemeinen Struktur IV 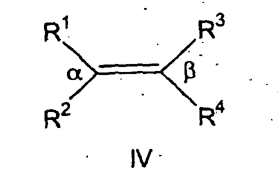 worin R¹, R², R³ und R⁴ wie oben definiert sind, in Gegenwart einer Säure umgesetzt wird.

Das erfindungsgemäße Verfahren wird bevorzugt in einer "Eintopf"-Reaktion durchgeführt, bei der je ein Heteroarylamin der allgemeinen Struktur II, je ein Aldehyd der allgemeinen Struktur III und je ein Olefin der allgemeinen Struktur IV gleichzeitig miteinander umgesetzt werden.

Das erfindungsgemäße Verfahren kann auch in semi- oder vollautomatisierter Form als Parallelsynthese einer Gruppe von erfindungsgemäßen Verbindungen der allgemeinen Struktur I durchgeführt werden.

Bei der eingesetzten Säure handelt es sich um eine anorganische oder organische Protonen- oder Lewis-Säure. Bevorzugt wird die Reaktion in Gegenwart einer organischen Säure, z.B. Essigsäure, Trifluoressigsäure oder Methansulfonsäure, insbesondere Trifluoressigsäure, durchgeführt.

Das erfindungsgemäße Herstellungsverfahren kann in jedem geeigneten Lösungsmittel durchgeführt werden, in dem die Reaktanten sich ausreichend lösen. Bevorzugt sind als Lösungsmittel organische Solventien, z.B. Dichlormethan oder insbesondere Acetonitril.

Die erfindungsgemäße Herstellung der erfindungsgemäßen Verbindungen der allgemeinen Struktur I erfolgt zweckmäßig bei einer Temperatur von 0 bis 100 °C, insbesondere bei 15 bis 40°C. Die Reaktionszeit beträgt vorzugsweise 15 Minuten bis 12 Stunden und kann den jeweiligen Erfordernissen angepaßt werden.

Die im erfindungsgemäßen Verfahren eingesetzten Heteroarylamine der allgemeinen Struktur II, die Aldehyde der allgemeinen Struktur III und die Olefine der allgemeinen Struktur IV sind käuflich erhältlich (von Acros, Geel; Avocado, Port of Heysham; Aldrich, Deisenhofen; Fluka, Seelze; Lancaster, Mülheim; Maybridge, Tintagel; Merck, Darmstadt; Sigma, Deisenhofen; TCl, Japan) oder können nach im Stand der Technik allgemein bekannten Verfahren hergestellt werden.

Die erfindungsgemäßen Verbindungen der allgemeinen Struktur I können sowohl als freie Base als auch als Salz isoliert werden. Die freie Base der Verbindung der allgemeinen Struktur I wird üblicherweise nach erfolgter Umsetzung gemäß dem oben dargelegten erfindungsgemäßen Verfahren und anschließender herkömmlicher Aufarbeitung erhalten. Die so gewonnene Base kann dann beispielsweise durch Umsetzung mit einer anorganischen oder organischen Säure, vorzugsweise mit Salzsäure, Bromwasserstoffsäure, Schwefelsäure, Phosphorsäure, Methansulfonsäure, p-Toluolsulfonsäure, Kohlensäure, Ameisensäure, Essigsäure, Oxalsäure, Bernsteinsäure, Weinsäure, Mandelsäure, Fumarsäure, Milchsäure, Citronensäure, Glutaminsäure oder Asparaginsäure, in das korrespondierende Salz überführt werden. Bei den gebildeten Salzen handelt es sich u.a. um Hydrochloride, Hydrobromide, Phosphate, Carbonate, Hydrogencarbonate, Formiate, Acetate, Oxalate, Succinate, Tartrate, Fumarate, Citrate und Glutaminate. Die besonders bevorzugte Hydrochloridbildung kann insbesondere auch durch Versetzen der in einem geeigneten organischen Lösungsmittel gelösten Base mit Trimethylsilylchlorid (TMSCI) herbeigeführt werden.

Soweit die Verbindungen der allgemeinen Struktur I in dem erfindungsgemäßen Herstellungsverfahren als Racemate oder als Mischungen ihrer verschiedenen Enantiomeren und/oder Diastereomeren erhalten werden, können diese Mischungen nach im Stand der Technik wohlbekannten Verfahren aufgetrennt werden. Geeignete Methoden sind u.a. chromatographische Trennverfahren, insbesondere Flüssigkeitschromatographie-Verfahren unter Normal- oder erhöhtem Druck, bevorzugt MPLC- und HPLC-Verfahren, sowie Verfahren der fraktionierten Kristallisation. Dabei können insbesondere einzelne Enantiomeren z.B. mittels HPLC an chiraler Phase oder mittels Kristallisation von mit chiralen Säuren, etwa (+)-Weinsäure, (-)-Weinsäure oder (+)-10-Camphersulfonsäure, gebildeten diastereomeren Salzen voneinander getrennt werden.

Darüber hinaus ist ein Arzneimittel, das mindestens eine der erfindungsgemäßen und wie oben definierten Verbindungen der allgemeinen Struktur I bzw. ihrer pharmazeutisch annehmbaren Salze umfaßt, Gegenstand der Erfindung. Dabei können die erfindungsgemäßen Verbindungen in dem erfindungsgemäßen Arzneimittel als isomerenreine, insbesondere enantiomerenreine bzw. diastereomerenreine, Verbindungen, aber auch als racemisches oder nicht-racemisches Gemisch vorliegen. Bevorzugt ist dabei, daß das Arzneimittel ein pharmazeutisch annehmbares Salz der erfindungsgemäßen Verbindungen enthält, insbesondere ein Hydrochlorid.

Ein weiterer Gegenstand der Erfindung ist die Verwendung mindestens einer erfindungsgemäßen Verbindung der allgemeinen Struktur I, einschließlich ihrer Diastereomeren oder Enantiomeren, auch als Racemate oder Enantiomerengemisch in Form ihrer freien Base oder eines mit einer physiologisch verträglichen Säure gebildeten Salzes, insbesondere des Hydrochloridsalzes, zur Herstellung eines Arzneimittels zur Behandlung von Schmerz. Die erfindungsgemäßen Verbindungen haben sich als analgetisch wirksam erwiesen.

Überraschenderweise hat es sich herausgestellt, daß die erfindungsgemäßen Verbindungen der allgemeinen Struktur I für weitere Indikationen, insbesondere zur Behandlung von Harninkontinenz, Juckreiz, Tinnitus aurium und/oder Diarrhoe, sehr geeignet sind. Ein weiterer Gegenstand der Anmeldung ist daher die Verwendung mindestens einer erfindungsgemäßen Verbindung der allgemeinen Struktur I einschließlich eines pharmazeutisch annehmbaren Salzes zur Herstellung eines Arzneimittels zur Behandlung von Haminkontinenz, Juckreiz, Tinnitus aurium und/oder Diarrhoe.

Darüber hinaus sind auch pharmazeutische Zusammensetzungen Gegenstand der vorliegenden Erfindung, die mindestens eine Verbindung der wie oben definierten allgemeinen Struktur I oder eines ihrer pharmazeutisch annehmbaren Salze und einen oder mehrere pharmazeutische Hilfsstoffe enthalten.

Die erfindungsgemäßen Arzneimittel und pharmazeutischen Zusammensetzungen können als flüssige, halbfeste oder feste Arzneiformen und in Form von z.B. Injektionslösungen, Tropfen, Säften, Sirupen, Sprays, Suspensionen, Granulaten, Tabletten, Pellets, transdermale therapeutische Systeme, Kapseln, Pflastern, Zäpfchen, Salben, Cremes, Lotionen, Gelen, Emulsionen oder Aerosolen vorliegen und verabreicht werden und enthalten neben mindestens einer erfindungsgemäßen Verbindung der allgemeinen Struktur I je nach galenischer Form pharmazeutische Hilfsstoffe, wie z.B. Trägermaterialien, Füllstoffe, Lösungsmittel, Verdünnungsmittel, oberflächenaktive Stoffe, Farbstoffe, Konservierungsstoffe, Sprengmittel, Gleitmittel, Schmiermittel, Aromen und/oder Bindemittel. Diese Hilfsstoffe können beispielsweise sein: Wasser, Ethanol, 2-Propanol, Glycerin, Ethylenglycol, Propylenglycol, Polyethylenglycol, Polypropylenglycol, Glucose, Fructose, Lactose, Saccharose, Dextrose, Melasse, Stärke, modifizierte Stärke, Gelatine, Sorbitol, Inositol, Mannitol, mikrokristalline Cellulose, Methylcellulose, Carboxymethylcellulose, Celluloseacetat, Schellack, Cetylalkohol, Polyvinylpyrrolidon, Paraffine, Wachse, natürliche und synthetische Gummis, Akaziengummi, Alginate, Dextran, gesättigte und ungesättigte Fettsäuren, Stearinsäure, Magnesiumstearat, Zinkstearat, Glycerylstearat, Natriumlaurylsulfat, genießbare Öle, Sesamöl, Kokusnußöl, Erdnußöl, Sojabohnenöl, Lecithin, Natriumlactat, Polyoxyethylen- und -propylenfettsäureester, Sorbitanfettsäureester, Sorbinsäure, Benzoesäure, Citronensäure, Ascorbinsäure, Tanninsäure, Natriumchlorid, Kaliumchlorid, Magnesiumchlorid, Calciumchlorid, Magnesiumoxid, Zinkoxid, Siliciumdioxid, Titanoxid, Titandioxid, Magnesiumsulfat, Zinksulfat, Calciumsulfat, Pottasche, Calciumphosphat, Dicalciumphosphat, Kaliumbromid, Kaliumiodid, Talkum, Kaolin, Pectin, Crospovidon, Agar und Bentonit.

Die Auswahl der Hilfsstoffe sowie die einzusetzenden Mengen derselben hängt davon ab, ob das Arzneimittel oral, subkutan, parenteral, intravenös, vaginal, pulmonal, intraperitoneal, transdermal, intramuskulär, nasal, buccal, rectal oder örtlich, zum Beispiel auf infektionen an der Haut, der Schleimhäute und an den Augen, appliziert werden soll. Für die orale Applikation eignen sich u.a. Zubereitungen in Form von Tabletten, Dragees, Kapseln, Granulaten, Tropfen, Säften und Sirupen, für die parenterale, topische und inhalative Applikation Lösungen, Suspensionen, leicht rekonstituierbare Pulver zur Inhalation sowie Sprays. Erfindungsgemäße Verbindungen der allgemeinen Struktur I in einem Depot in gelöster Form oder in einem Pflaster, gegebenenfalls unter Zusatz von die Hautpenetration fördernden Mitteln, sind geeignete perkutane Applikationszubereitungen. Rektal, transmucosal, parenteral, oral oder perkutan anwendbare Zubereitungsformen können die erfindungsgemäßen Verbindungen der allgemeinen Struktur I verzögert freisetzen.

Die Herstellung der erfindungsgemäßen Arzneimittel und pharmazeutischen Zusammensetzungen erfolgt mit Hilfe von im Stand der Technik der pharmazeutischen Formulierung wohlbekannten Mitteln, Vorrichtungen, Methoden und Verfahren, wie sie beispielsweise in "Remington's Pharmaceutical Sciences", Hrsg. A.R. Gennaro, 17. Ed., Mack Publishing Company, Easton, Pa. (1985), insbesondere in Teil 8, Kapitel 76 bis 93, beschrieben sind.

So kann z.B. für eine feste Formulierung, wie eine Tablette, der Wirkstoff des Arzneimittels, d.h. eine Verbindung der allgemeinen Struktur I oder eines ihrer pharmazeutisch annehmbaren Salze, mit einem pharmazeutischen Träger, z.B. herkömmlichen Tabletteninhaltsstoffen, wie Maisstärke, Lactose, Saccharose, Sorbitol, Talkum, Magnesiumstearat, Dicalciumphosphat oder pharmazeutisch akzeptable Gummis, und pharmazeutischen Verdünnungsmitteln, wie z.B. Wasser, granuliert werden, um eine feste Zusammensetzungzu bilden, die eine erfindungsgemäße Verbindung oder ein pharmazeutisch annehmbares Salz davon in homogener Verteilung enthält. Unter einer homogenen Verteilung wird hier verstanden, daß der Wirkstoff gleichmäßig über die gesamte Zusammensetzung verteilt ist, so daß diese ohne weiteres in gleich wirksame Einheitsdosis-Formen, wie Tabletten, Pillen oder Kapseln, unterteilt werden kann. Die feste Zusammensetzungwird anschließend in Einheitsdosis-Formen unterteilt. Die Tabletten oder Pillen des erfindungsgemäßen Arzneimittels bzw. der erfindungsgemäßen Zusammensetzungen können auch überzogen oder auf andere Weise kompoundiert werden, um eine Dosisform mit verzögerter Freisetzung bereitzustellen. Geeignete Beschichtungsmittel sind u.a. polymere Säuren und Mischungen von polymeren Säuren mit Materialien wie z.B. Schellack, Cetylalkohol und/oder Celluloseacetat.

Die an den Patienten zu verabreichende Wirkstoffmenge variiert und ist abhängig vom Gewicht, dem Alter und der Krankheitsgeschichte des Patienten, sowie von der Applikationsart, der Indikation und dem Schweregrad der Erkrankung. Üblicherweise werden 0,1 bis 5000 mg/kg, insbesondere 1 bis 500 mg/kg, vorzugsweise 2 bis 250 mg/kg Körpergewicht wenigstens einer erfindungsgemäßen Verbindung der allgemeinen Struktur I appliziert.

Die nachfolgenden Beispiele dienen zur näheren Erläuterung der vorliegenden Erfindung.

### Beispiele

Die eingesetzten Chemikalien und Lösungsmittel wurden kommerziell von einem der folgenden Anbieter erworben: Acros, Geel; Avocado, Port of Heysham; Aldrich, Deisenhofen; Fluka, Seelze; Lancaster, Mülheim; Maybridge, Tintagel; Merck, Darmstadt; Sigma, Deisenhofen; TCl, Japan; oder nach allgemeinen im Stand der Technik bekannten Verfahren hergestellt.

Die chromatographische Reinigung erfolgte an einer HPLC-RP-18-Säule der Fa. Macherey-Nagel; Material NUCLEOSIL 100-3 C₁₈-HD. ca. 100 mm (VarioPrep), Innendurchmesser 21 mm; Laufmittel Wasser/Methanol, Gradient: 50-100 % in ca. 18 min., Fluß: 10 ml/min.; Detektion: UV, Beckman 168 PDA.

### Allgemeine Arbeitsvorschrift AAV (Semiautomatisierte Synthese)

Ein Rundbodenröhrchen aus Glas (Durchmesser 16 mm, Länge 125 mm) mit Gewinde wurde mit einem Rührer versehen und mit einem Schraubdeckel mit Septum verschlossen. Das Röhrchen wurde in den auf 20 °C temperierten Rührblock gestellt. Anschließend wurden nacheinander die folgenden Reagenzien hinzupipettiert:
1 ml einer Lösung aus Trifluoressigsäure, 0,1 M, und
Heteroarylaminkomponente II, 0,1 M, in Acetonitril;
1 ml einer 0,11 M Lösung des Aldehyds III in Acetonitril;
1 ml einer 0,3 M Lösung des Olefins IV in Acetonitril.

Das Reaktionsgemisch wurde bei 20 °C in einem der Rührblöcke 10 h lang gerührt. Danach wurde die Reaktionslösung abfiltriert. Das Röhrchen wurde dabei zweimal mit je 1,5 ml einer 7,5% NaHCO₃-Lösung gespült.

Das Rack mit den Proben wurde manuell auf die Aufarbeitungsanlage gestellt. Das Reaktionsgemisch wurde auf einem Vortexer mit 2 ml Ethylacetat versetzt und geschüttelt. Zur Ausbildung der Phasengrenze wurde in der Zentrifuge kurz zentrifugiert. Die Phasengrenze wurde optisch detektiert und die organische Phase abpipettiert. Im nächsten Schritt wurde die wäßrige Phase erneut mit 2 ml Ethylacetat versetzt, geschüttelt, zentrifugiert und die organische Phase abpipettiert. Die vereinigten organischen Phasen wurden über 2,4 g MgSO₄ (granuliert) getrocknet. Das Lösungsmittel wurde in einer Vakuumzentrifuge entfernt.

Jede Probe wurde mit ESI-MS und/oder NMR charakterisiert.
Massenspektrometrische Untersuchungen (ESI-MS) wurden mit einem Massenspektrometer der Fa. Finnegan, LCQ Classic durchgeführt. ¹H-NMR-Untersuchungen der erfindungsgemäßen Verbindungen wurden mit einem 300 MHz DPX Advance NMR-Gerät der Fa. Bruker durchgeführt.

Nach der angegebenen AAV wurden erfindungsgemäße Beispiele (*) sowie Referenzbeispiele 1-131 (s. Tabelle 1) hergestellt. Die Beispiele 80 bis 89 wurden mittels reversed-phase-(RP-)
HPLC aufgereinigt.

| **Beispiel** | **berechnete Masse** | **gefundene Masse** | **Verbindung** |
|---|---|---|---|
| 1 | 400,78 | 401,2/403,2 | 9-Chlor-4-(4-methoxy-phenyl)-3-methyl-7-trifluormethyl-3,4-dihydro-2H-pyrido[1,2-a]pyrimidin-2-carbonsäure |
| 2 | 428,83 | 429,2/430,1 | 9-Chlor-4-(4-methoxy-phenyl)-3-methyl-7-trifluormethyl-3,4-dihydro-2H-pyrido[1,2-a]pyrimidin-2-carbonsäureethylester |
| 3 | 411,28 | 411,1/413,1 | 7,9-Dichlor-4-(3,4-dimethoxy-phenyl)-3,4-dihydro-2H-pyrido[1,2-a]pyrimidin-2-carbonsäureethylester |
| 4 | 500,19 | 499,1/501,0/503,0 | 7,9-Dibrom-4-(3,4-dimethoxy-phenyl)-3,4-dihydro-2H-pyrido[1,2-a]pyrimidin-2-carbonsäureethylester |
| 5 | 387,39 | 388,2 | 4-(3,4-Dimethoxy-phenyl)-7-nitro-3,4-dihydro-2H-pyrido[1,2-a]pyrimidin-2-carbonsäureethylester |
| 6 | 381,25 | 381,1/383,0 | 7,9-Dichlor-4-(4-methoxy-phenyl)-3,4-dihydro-2H-pyrido[1,2-a]pyrimidin-2-carbonsäureethylester |
| 7 | 357,36 | 358,2 | 4-(4-Methoxy-phenyl)-7-nitro-3,4-dihydro-2H-pyrido[1,2-a]pyrimidin-2-carbonsäureethylester |
| 8 | 383,23 | 383,2/385,0 | 7,9-Dichlor-4-(3,4-dimethoxy-phenyl)-3,4-dihydro-2H-pyrido[1,2-a]pyrimidin-2-carbonsäure |
| 9 | 486,16 | 485,1/487,0/488,9 | 7,9-Dibrom-4-(3,4-dimethoxy-phenyl)-6-methyl-3,4-dihydro-2H-pyrido[1,2-a]pyrimidin-2-carbonsäure |
| 10 | 359,33 | 360,1 | 4-(3,4-Dimethoxy-phenyl)-7-nitro-3,4-dihydro-2H-pyrido[1,2-a]pyrimidin-2-carbonsäure |
| 11 | 353,2 | 353,1/355,0 | 7,9-Dichlor-4-(4-methoxy-phenyl)-3,4-dihydro-2H-pyrido[1,2-a]pyrimidin-2-carbonsäure |
| 12 | 329,31 | 330,1 | 4-(4-Methoxy-phenyl)-7-nitro-3,4-dihydro-2H-pyrido[1,2-a]pyrimidin-2-carbonsäure |
| 13 | 408,21 | 408,1/410,1 | 7-Brom-4-(4-methoxy-phenyl)-9-nitro-3,4-dihydro-2H-pyrido[1,2-a]pyrimidin-2-carbonsäure |
| 14 | 450,27 | 450,2/452,1 | 7,9-Dichlor-4-(3,4-dimethoxy-phenyl)-2-(5-nitro- furan-2-yl)-3,4-dihydro-2H-pyrido[1,2-a]pyrimidin |
| 15 | 539,18 | 538,1/540,1/542,0 | 7,9-Dibrom-4-(3,4-dimethoxy-phenyl)-2-(5-nitro-furan-2-yl)-3,4-dihydro-2H-pyrido[1,2-a]pyrimidin |
| 16 | 426,38 | 427,2 | 4-(3,4-Dimethoxy-phenyl)-7-nitro-2-(5-nitro-furan-2-yl)-3,4-dihydro-2H-pyrido[1,2-a]pyrimidin |
| 17 | 420,25 | 420,2/422,1 | 7,9-Dichlor-4-(4-methoxy-phenyl)-2-(5-nitro-furan-2-yl)-3,4-dihydro-2H-pyrido[1,2-a]pyrimidin |
| 18 | 419,35 | 419,3/421,1 | 2-[7,9-Dichlor-4-(2,4-dimethyl-phenyl)-3,4-dihydro-2H-pyrido[1,2-a]pyrimidin-2-yl]-cyclopropancarbonsäureethylester |
| 19 | 452,9 | 453,3 | 2-[9-Chlor-4-(2,4-dimethyl-phenyl)-7-trifluormethyl-3,4-dihydro-2H-pyrido[1,2-a]pyrimidin-2-yl]-cyclopropancarbonsäureethylester |
| 20 | 540,26 | 539,3/541,1/543,1 | 2-[7,9-Dibrom-4-(3,4-dimethoxy-phenyl)-3,4-dihydro-2H-pyrido[1,2-a]pyrimidin-2-yl]-cyclopropancarbonsäureethylester |
| 21 | 421,32 | 421,1/423,1 | 2-[7,9-Dichlor-4-(4-methoxy-phenyl)-3,4-dihydro-2H-pyrido[1,2-a]pyrimidin-2-yl]-cyclopropancarbonsäureethylester |
| 22 | 510,23 | 509,1/511,0/513,0 | 2-[7,9-Dibrom-4-(4-methoxy-phenyl)-3,4-dihydro-2H-pyrido[1,2-a]pyrimidin-2-yl]-cyclopropancarbonsäureethylester |
| 23 | 454,87 | 455,2/456,0/457,1 | 2-[9-Chlor-4-(4-methoxy-phenyl)-7-trifluormethyl-3,4-dihydro-2H-pyrido[1,2-a]pyrimidin-2-yl]-cyclopropancarbonsäureethylester |
| 24 | 397,43 | 398,2 | 2-[4-(4-Methoxy-phenyl)-7-nitro-3,4-dihydro-2H-pyrido[1,2-a]pyrimidin-2-yl]-cyclopropancarbonsäureethylester |
| 25 | 357,36 | 358,2 | 4-(4-Hydroxy-phenyl)-3-methyl-7-nitro-3,4-dihydro-2H-pyrido[1,2-a]pyrimidin-2- carbonsäureethylester |
| 26 | 355,24 | 355,1/357,0 | 7,9-Dichlor-4-phenylsulfanyl-3,4-dihydro-2H-pyrido[1,2-a]pyrimidin-2-carbonsäure |
| 27 | 331,34 | 332,1 | 7-Nitro-4-phenylsulfanyl-3,4-dihydro-2H-pyrido[1,2-a]pyrimidin-2-carbonsäure |
| 28 | 383,23 | 383,1/385,1 | 7,9-Dichlor-4-(4-hydroxy-3-methoxy-phenyl)-3-methyl-3,4-dihydro-2H-pyrido[1,2-a]pyrimidin-2-carbonsäure |
| 29 | 472,14 | 471,0/473,0/475,0 | 7,9-Dibrom-4-(4-hydroxy-3-methoxy-phenyl)-3-methyl-3,4-dihydro-2H-pyrido[1,2-a]pyrimidin-2-carbonsäure |
| 30 | 486,16 | 485,0/487,0/488,9 | 7,9-Dibrom-4-(4-hydroxy-3-methoxy-phenyl)-3,6-dimethyl-34-dihydro-2H-pyrido[1,2-a]pyrimidin-2-carbonsäure |
| 31 | 416,78 | 417,1/419,1 | 9-Chlor-4-(4-hydroxy-3-methoxy-phenyl)-3-methyl-7-trifluormethyl-3,4-dihydro-2H-pyrido[1,2-a]pyrimidin-2-carbonsäure |
| 32 | 367,23 | 367,1/369,1 | 7,9-Dichlor-4-(4-methoxy-phenyl)-3-methyl-3,4-dihydro-2H-pyrido[1,2-a]pyrimidin-2-carbonsäure |
| 33 | 456,14 | 455,0/457,0/459,0 | 7,9-Dibrom-4-(4-methoxy-phenyl)-3-methyl-3,4-dihydro-2H-pyrido[1,2-a]pyrimidin-2-carbonsäure |
| 34 | 470,17 | 469,0/471,0/472,9 | 7,9-Dibrom-4-(4-methoxy-phenyl)-3,6-dimethyl-3,4-dihydro-2H-pyrido[1,2-a]pyrimidin-2-carbonsäure |
| 35 | 400,78 | 401,1/403,1 | 9-Chlor-4-(4-methoxy-phenyl)-3-methyl-7-trifluormethyl-3,4-dihydro-2H-pyrido[12-a]pyrimidin-2-carbonsäure |
| 36 | 343,33 | 344,2 | 4-(4-Methoxy-phenyl)-3-methyl-7-nitro-3,4-dihydro-2H-pyrido[1,2-a]pyrimidin-2-carbonsäure |
| 37 | 422,23 | 422,1/424,0 | 7-Brom-4-(4-methoxy-phenyl)-3-methyl-9-nitro-34-dihydro-2H-pyrido[12-a]pyrimidin-2-carbonsäure |
| 38 | 353,2 | 353,1/355,1 | 7,9-Dichlor-4-(4-hydroxy-phenyl)-3-methyl-3,4-dihydro-2H-pyrido[1,2-a]pyrimidin-2-carbonsäure |
| 39 | 456,14 | 455,0/457,0/459,0 | 7,9-Dibrom-4-(4-hydroxy-phenyl)-3,6-dimethyl-3,4-dihydro-2H-pyrido[1,2-a]pyrimidin-2-carbonsäure |
| 40 | 383,23 | 383,1/385,1 | 7,9-Dichlor-4-(3,4-dimethoxy-phenyl)-3,4-dihydro-2H-pyrido[1,2-a]pyrimidin-2-carbonsäure |
| 41 | 472,14 | 471,0/473,0/475,0 | 7,9-Dibrom-4-(3,4-dimethoxy-phenyl)-3,4-dihydro-2H-pyrido[1,2-a]pyrimidin-2-carbonsäure |
| 42 | 486,16 | 485,0/487,0/489,0 | 7,9-Dibrom-4-(3,4-dimethoxy-phenyl)-6-methyl-3,4-dihydro-2H-pyrido[1,2-a]pyrimidin-2-carbonsäure |
| 43 | 416,78 | 417,1 | 9-Chlor-4-(3,4-dimethoxy-phenyl)-7-trifluormethyl-3,4-dihydro-2H-pyrido[1,2-a]pyrimidin-2- carbonsäure |
| 44 | 359,33 | 360,2 | 4-(3,4-Dimethoxy-phenyl)-7-nitro-3,4-dihydro-2H-pyrido[1,2-a]pyrimidin-2-carbonsäure |
| 45 | 285,3 | 286,2 | 4-(4-Methoxy-phenyl)-3,4-dihydro-2H-pyrazino[1,2-a]pyrimidin-2-carbonsäure |
| 46 | 353,2 | 353,1/355,1 | 7,9-Dichlor-4-(4-methoxy-phenyl)-3,4-dihydro-2H-pycido[1,2-a]pyrimidin-2-carbonsäure |
| 47 | 442,11 | 441,0/443,0/445,0 | 7,9-Dibrom-4-(4-methoxy-phenyl)-3,4-dihydro-2H-pyrido[1,2-a]pyrimidin-2-carbonsäure |
| 48 | 456,14 | 455,0/457,0/459,0 | 7,9-Dibrom-4-(4-methoxy-phenyl)-6-methyl-3,4-dihydro-2H-pyrido[1,2-a]pyrimidin-2-carbonsäure |
| 49 | 386,75 | 387,1 | 9-Chlor-4-(4-methoxy-phenyl)-7-trifluormethyl-3,4-dihydro-2H-pyrido[1,2-a]pyrimidin-2-carbonsäure |
| 50 | 329,31 | 330,1 | 4-(4-Methoxy-phenyl)-7-nitro-3,4-dihydro-2H-pyrido[1,2-a]pyrimidin-2-carbonsäure |
| 51* | 443,32 | 441,2/443,1/445,1 | [7,9-Dichlor-4-(4-hydroxy-3-methoxy-phenyl)-3-methyl-3,4-dihydro-2H-pyrido[1,2-a]pyrimidin-2- yl]-phenyl-methanon |
| 52 | 532,24 | 533,0 | [7,9-Dibrom-4-(4-hydroxy-3-methoxy-phenyl)-3-methyl-3,4-dihydro-2H-pyrido[1,2-a]pyrimidin-2-yl]-phenyl-methanon |
| 53 | 546,26 | 545,0/547,0/549,0 | [7,9-Dibrom-4-(4-hydroxy-3-methoxy-phenyl)-3,6-dimethyl-3,4-dihydro-2H-pyrido[1,2-a]pyrimidin-2-yl]-phenyl-methanon |
| 54 | 419,43 | 420,2 | [4-(4-Hydroxy-3-methoxy-phenyl)-3-methyl-7-nitro-3,4-dihydro-2H-pyrido[1,2-a]pyrimidin-2-yl]-phenyl-methanon |
| 55 | 403,43 | 404,2 | [4-(4-Methoxy-phenyl)-3-methyl-7-nitro-3,4-dihydro-2H-pyrido[1,2-a]pyrimidin-2-yl]-phenyl-methanon |
| 56* | 516,24 | 515,0/517,0/519,0 | [7,9-Dibrom-4-(4-hydroxy-phenyl)-3,6-dimethyl-3,4-dihydro-2H-pyrido[1,2-a]pyrimidin-2-yl]-phenyl-methanon |
| 57 | 443,32 | 443,11445,1 | [7,9-Dichlor-4-(3,4-dimethoxy-phenyl)-3,4-dihydro-2H-pyrido[1,2-a]pyrimidin-2-yl]-phenyl-methanon |
| 58 | 532,24 | 531,1/533,1 | [7,9-Dibrom-4-(3,4-dimethoxy-phenyl)-3,4-dihydro-2H-pyrido[1,2-a]pyrimidin-2-yl]-phenyl-methanon |
| 59 | 546,26 | 545,1/547,0 | [7,9-Dibrom-4-(3,4-dimethoxy-phenyl)-6-methyl-3,4-dihydro-2H-pyrido[1,2-a]pyrimidin-2-yl]-phenyl-methanon |
| 60 | 476,88 | 477,1 | [9-Chlor-4-(3,4-dimethoxy-phenyl)-7-trifluormethyl-3,4-dihydro-2H-pyrido[1,2-a]pyrimidin-2-yl]-phenyl-methanon |
| 61 | 419,43 | 420,2 | [4-(3,4-Dimethoxy-phenyl)-7-nitro-3,4-dihydro-2H-pyrido[1,2-a]pyrimidin-2-yl]-phenyl-methanon |
| 62 | 389,41 | 390,2 | [4-(4-Methoxy-phenyl)-7-nitro-3,4-dihydro-2H-pyrido[1,2-a]pyrimidin-2-yl]-phenyl-methanon |
| 63 | 303,18 | 303,1/305,1 | 2-(7,9-Dichlor-2-cyclopropyl-3,4-dihydro-2H-pyrido[1,2-a]pyrimidin-4-yloxy)-ethanol |
| 64 | 392,1 | 391,0/393,01395,0 | 2-(7,9-Dibrom-2-cyclopropyl-3,4-dihydro-2H- pyrido[1,2-a]pyrimidin-4-yloxy)-ethanol |
| 65 | 336,74 | 337,1/339,1 | 2-(9-Chlor-2-cyclopropyl-7-trifluormethyl-3,4-dihydro-2H-pyrido[1,2-a]pyrimidin-4-yloxy)-ethanol |
| 66 | 279,29 | 280,1 | 2-(2-Cyclopropyl-7-nitro-3,4-dihydro-2H-pyrido[1,2-a]pyrimidin-4-yloxy)-ethanol |
| 67 | 412,83 | 413,1 | 9-Chlor-2-cyclopropyl-4-(3,4-dimethoxy-phenyl)-7-trifluormethyl-3,4-dihydro-2H-pyrido[1,2-a]pyrimidin |
| 68 | 438,17 | 439,0 | 7,9-Dibrom-2-cyclopropyl-4-(4-methoxy-phenyl)-3,4-dihydro-2H-pyrido[1,2-a]pyrimidin |
| 69 | 382,81 | 383,1/385,1 | 9-Chlor-2-cyclopropyl-4-(4-methoxy-phenyl)-7-trifluormethyl-3,4-dihydro-2H-pyrido[1,2-a]pyrimidin |
| 70 | 325,36 | 326,2 | 2-Cyclopropyl-4-(4-methoxy-phenyl)-7-nitro-3,4-dihydro-2H-pyrida[1,2-a]pyrimidin |
| 71 | 345,26 | 345,1/347,1 | 2-(7,9-Dichlor-2-cyclohexyl-3,4-dihydro-2H-pyrido[1,2-a]pyrimidin-4-yloxy)-ethanol |
| 72 | 434,18 | 433,1/435,1/437,0 | 2-(7,9-Dibrom-2-cyclohexyl-3,4-dihydro-2H-pyrido[1,2-a]pyrimidin-4-yloxy)-ethanol |
| 73 | 448,2 | 447,1/449,0/451,0 | 2-(7,9-Dibrom-2-cyclohexyl-6-methyl-3,4-dihydro-2H-pyrido[1,2-a]pyrimidin-4-yloxy)-ethanol |
| 74 | 378,82 | 379,2/381,2 | 2-(9-Chlor-2-cyclohexyl-7-trifluormethyl-3,4-dihydro-2H-pyrido[1,2-a]pyrimidin-4-yloxy)-ethanol |
| 75 | 321,37 | 322,2 | 2-(2-Cyclohexyl-7-nitro-3,4-dihydro-2H-pyrido[1,2-a]pyrimidin-4-yloxy)-ethanol |
| 76 | 421,36 | 421,1/423,1 | 7,9-Dichlor-2-cyclohexyl-4-(3,4-dimethoxy-phenyl)-3,4-dihydro-2H-pyrido[1,2-a]pyrimidin |
| 77* | 391,34 | 391,1/393,1 | 7,9-Dichlor-2-cyclohexyl-4-(4-methoxy-phenyl)-3,4-dihydro-2H-pyrido[1,2-a]pyrimidin |
| 78* | 480,25 | 479,1/481,1/483,0 | 7,9-Dibrom-2-cyclohexyl-4-(4-methoxy-phenyl)-3,4-dihydro-2H-pyrido[1,2-a]pyrimidin |
| 79 | 424,89 | 425,2/427,2 | 9-Chlor-2-cyclohexyl-4-(4-methoxy-phenyl)-7- trifluormethyl-3,4-dihydro-2H-pyrido[1,2-a]pyrimidin |
| 80 | 484,19 | 485,0/487,0 | 7,9-Dibrom-4-(4-methoxy-phenyl)-3-methyl-3,4-dihydro-2H-pyrido[1,2-a]pyrimidin-2-carbonsäureethylester |
| 81 | 456,14 | 457,4/459,5 | 7,9-Dibrom-4-(4-methoxy-phenyl)-3-methyl-3,4-dihydro-2H-pyrido[1,2-a]pyrimidin-2-carbonsäure |
| 82 | 523,19 | 522,4/524,3/526,2 | 7,9-Dibrom-4-(4-methoxy-phenyl)-3-methyl-2-(5-nitro-furan-2-yl)-3,4-dihydro-2H-pyrido[1,2-a]pyrimidin |
| 83 | 524,26 | 525,2 | 2-[7,9-Dibrom-4-(4-methoxy-phenyl)-3-methyl-3,4-dihydro-2H-pyrido[1,2-a]pyrimidin-2-yl]-cyclopropancarbonsäureethylester |
| 84* | 553,21 | 554,2/555,2/556,2 | 7,9-Dibrom-4-(3,4-dimethoxy-phenyl)-6-methyl-2-(5-nitro-furan-2-yl)-3,4-dihydro-2H-pyrido[1,2-a]pyrimidin |
| 85 | 396,35 | 397,1 | 4-(4-Methoxy-phenyl)-7-nitro-2-(5-nitro-furan-2-yl)-3,4-dihydro-2H-pyrido[1,2-a]pyrimidin |
| 86 | 508,26 | 509,1 | 2-[7,9-Dibrom-4-(2,4-dimethyl-phenyl)-3,4-dihydro-2H-pyrido[1,2-a]pyrimidin-2-yl]-cyclopropan-carbonsäureethylester |
| 87 | 554,28 | 553,2/555,1/557,1 | 2-[7,9-Dibrom-4-(3,4-dimethoxy-phenyl)-6-methyl-3,4-dihydro-2H-pyrido[1,2-a]pyrimidin-2-yl]-cyclopropancarbonsäureethylester |
| 88 | 484,9 | 485,5 | 2-[9-Chlor-4-(3,4-dirnethoxy-phenyl)-7-trifluormethyl-3,4-dihydro-2H-pyrido[1,2-a]pyrimidin-2-yl]-cyclopropancarbonsäureethylester |
| 89* | 524,26 | 525,1/527,2 | 2-[7,9-Dibrom-4-(4-methoxy-phenyl)-6-methyl-3,4-dihydro-2H-pyrido[1,2-a]pyrimidin-2-yl)-cyclopropancarbonsäureethylester |
| 90 | 399,42 | 400,2 | 2-[4-(4-Fluor-phenyl)-4-methyl-7-nitro-3,4-dihydro-2H-pyrido[1,2-a]pyrimidin-2-yl]-cyclopropancarbonsäureethylester |
| 91 | 393,51 | 291,3 (M+H-OBenzyl) | 9-Benzyloxy-4-phenyl-2-pyridin-2-yl-3,4-dihydro-2H-pyrido[1,2-a]pyrimidin |
| 92 | 346,41 | 347,3 | 8-Methyl-7-nitro-4-phenyl-2-pyridin-2-yl-3,4-dihydro-2H-pyrido[1,2-a]pyrimidin |
| 93 | 356,28 | 356,2/358,2 | 7,9-Dichlor-4-phenyl-2-pyridin-2-yl-3,4-dihydro-2H-pyrido[1,2-a]pyrimidin |
| 94 | 389,83 | 390,3/392,2 | 9-Chlor-4-phenyl-2-pyridin-2-yl-7-trifluormethyl-3,4-dihydro-2H-pyrido[1,2-a]pyrimidin |
| 95 | 346.41 | 347,1 | 8-Methyl-9-nitro-4-phenyl-2-pyridin-2-yl-3,4-dihydro-2H-pyrido[1,2-a]pyrimidin |
| 96 | 332,38 | 333,2 | 7-Nitro-4-phenyl-2-pyridin-2-yl-3,4-dihydro-2H-pyrido[1,2-a]pyrimidin |
| 97 | 332,38 | 333,2 | 9-Nitro-4-phenyl-2-pyridin-2-yl-3,4-dihydro-2H-pyrido[1,2-a]pyrimidin |
| 98 | 411,28 | 413,1/416,2 | 7-Brom-9-nitro-4-phenyl-2-pyridin-2-yl-3,4-dihydro-2H-pyrido[1,2-a]pyrimidin |
| 99 | 459,2 | 458,1/460,0/462,0 | 7,9-Dibrom-6-methyl-4-phenyl-2-pyridin-2-yl-3,4-dihydro-2H-pyrido[1,2-a]pyrimidin |
| 100 | 445,18 | 444,2/446,1/448,0 | 7,9-Dibrom-4-phenyl-2-pyridin-2-yl-3,4-dihydro-2H-pyrido[1,2-a]pyrimidin |
| 101 | 363,44 | 364.2 | 4-Phenylsulfanyl-2-pyridin-2-yl-3,4-dihydro-2H-pyrido[1,2-a]pyrimidin-9-carbonsäure |
| 102 | 333.45 | 334,2 | 7-Methyl-4-phenylsulfanyl-2-pyridin-2-yl-3,4-dihydro-2H-pyrido[1,2-a]pyrimidin |
| 103 | 425,55 | 426,2 | 9-Benzyloxy-4-phenylsulfanyl-2-pyridin-2-yl-3,4-dihydro-2H-pyrido[1,2-a]pyrimidin |
| 104 | 378,45 | 379,2 | 8-Methyl-7-nitro-4-phenylsulfanyl-2-pyridin-2-yl-3,4-dihydro-2H-pyrido[1,2-a]pyrimidin |
| 105 | 398,87 | 399,1/400,0 | 6-Chlor-9-nitro-4-phenylsulfanyl-2-pyridin-2-yl-3,4-dihydro-2H-pyrido[1,2-a]pyrimidin |
| 106 | 388,32 | 388,2/390,1 | 7,9-Dichlor-4-phenylsulfanyl-2-pyridin-2-yl-3,4-dihydro-2H-pyrido[1,2-a]pyrimidin |
| 107 | 421,88 | 422,2/424,1/425,1 | 9-Chlor-4-phenylsulfanyl-2-pyridin-2-yl-7-trifluormethyl-3,4-dihydro-2H-pyrido[1,2- a]pyrimidin |
| 108 | 378,45 | 379,2 | 8-Methyl-9-nitro-4-phenylsulfanyl-2-pyridin-2-yl-3,4-dihydro-2H-pyrido[1,2-a]pyrimidin |
| 109 | 364,42 | 365,2 | 7-Nitro-4-phenylsulfanyl-2-pyridin-2-yl-3,4-dihydro-2H-pyrido[1,2-a]pyrimidin |
| 110 | 398,33 | 398,1/400,1/401,1 | 7-Brom-4-phenylsulfanyl-2-pyridin-2-yl-3,4-dihydro-2H-pyrido[1,2-a]pyrimidin |
| 111 | 364,42 | 365,2 | 9-Nitro-4-phenylsulfanyl-2-pyridin-2-yl-3,4-dihydro-2H-pyrido[1,2-a]pyrimidin |
| 112 | 412,36 | 414,1/415,1 | 7-Brom-9-methyl-4-phenylsulfanyl-2-pyridin-2-yl-3,4-dihydro-2H-pyrido[1,2-a]pyrimidin |
| 113 | 412,36 | 414,1 | 4-Phenylsulfanyl-2-pyridin-2-yl-3,4-dihydro-2H-pyrido[1,2-a]pyrimidin-9-ol |
| 114 | 335,43 | 353,2/356,1 (M+H₂O) | 7-Chlor-4-phenylsulfanyl-2-pyridin-2-yl-3,4-dihydro-2H-pyrido[1,2-a]pyrimidin |
| 115 | 335,43 | 336,2 | 4-Phenylsulfanyl-6-propyl-2-pyridin-2-yl-3,4-dihydro-2H-pyrido[1,2-a]pyrimidin |
| 116 | 491,25 | 492,1 | 7,9-Dibrom-6-methyl-4-phenylsulfanyl-2-pyridin-2-yl-3,4-dihydro-2H-pyrido[1,2-a]pyrimidin |
| 117 | 387,43 | 388,2 | 4-Phenylsulfanyl-2-pyridin-2-yl-7-trifluormethyl-3,4-dihydro-2H-pyrido[1,2-a]pyrimidin |
| 118 | 477,23 | 476,11478,0/480,0 | 7,9-Dibrom-4-phenylsulfanyl-2-pyridin-2-yl-3,4-dihydro-2H-pyrido[1,2-a]pyrimidin |
| 119 | 385,49 | 386,2 | 1-Phenylsulfanyl-3-pyridin-2-yl-2,3-dihydro-1H-pyrimido[1,2-a]chinolin-10-ol |
| 120 | 289,39 | 288,4 | 2-Methyl-6-pyridin-2-yl-7,10-methano-6a,7,10,10a-tetrahydro-6H-pyrido[1,2-a]chinazolin |
| 121 | 344,26 | 344,3/361,2/363,2 (M+H₂O) | 2,4-Dichlor-6-pyridin-2-yl-7,10-methano-6a,7,10,10a-tetrahydro-6H-pyrido[1,2-a]chinazolin |
| 122 | 377,81 | 378,3/395,2/397,1 (M+H₂O) | 4-Chlor-6-pyridin-2-yl-2-trifluormethyl-7,10-methano-6a,7,10,10a-tetrahydro-6H-pyrido[1,2-a]chinazolin |
| 123 | 320,35 | 338,2 (M+H₂O) | 2-Nitro-6-pyridin-2-yl-7,10-methano-6a,7,10,10a-tetrahydro-6H-pyrido[1,2-a]chinazolin |
| 124 | 320,35 | 338,2 (M+H₂O) | 4-Nitro-6-pyridin-2-yl-7,10-methano-6a,7,10,10a-tetrahydro-6H-pyrido[1,2-a]chinazolin |
| 125 | 309,81 | 307,4/327,2 (M+H₂O) | 2-Chlor-6-pyridin-2-yl-7,10-methano-6a,7,10,10a-tetrahydro-6H-pyrido[1,2-a]chinazolin |
| 126 | 317,45 | 335,3 (M+H₂O) | 1-Propyl-6-pyridin-2-yl-7,10-methano-6a,7,10,10a-tetrahydro-6H-pyrido[1,2-a]chinazolin |
| 127 | 447,18 | 446,2/448,2/450,1 | 2,4-Dibrom-1-methyl-6-pyridin-2-yl-7,10-methano-6a,7,10,10a-tetrahydro-6H-pyrido[1,2-a]chinazolin |
| 128 | 343,36 | 361,2 (M+H₂O) | 6-Pyridin-2-yl-2-trifluormethyl-7,10-methano-6a,7,10,10a-tetrahydro-6H-pyrido[1,2-a]chinazolin |
| 129 | 341,43 | 324,1 | 1,4-Methano-5-pyridin-2-yl-1,4a,5,12c-tetrahydro-4H-6,12b-diaza-benzo[c]phenathren-12-ol |
| 130 | 325,42 | 324,2 | 1,4-Methano-5-pyridin-2-yl-1,4a,5,12c-tetrahydro-4H-6,12b-diaza-benzo[c]phenathren |
| 131 | 325,42 | 324,2 | 6-Pyridin-2-yl-7,10-methano-6a,7,10,10a-tetrahydro-6H-isochino[2,1-a]chinazolin |

### Pharmakologische Untersuchungen

### Untersuchungen zur Noradrenalin-Wiederaufnahmehemmung (NA-Uptakehemmung)

Um diese in vitro Studien durchfühern zu können, werden Synaptosomen aus Rattenhimarealen frisch isoliert. Es findet jeweils eine sogenannte "P₂"-Fraktion Verwendung, die nach der Vorschrift von Gray und Whittaker (E.G. Gray und V. P. Whittaker (1962) J. Anat. 76, 79-88) präpariert wird. Für den NA-Uptake werden diese vesikulären Partikel aus dem Hypothalamus männlicher Rattengehirne isoliert.

Folgende Kenndaten wurden für den NA-Transporter ermittelt:
NA-Uptake: Km = 0,32 ± 0,11 µM
(Jeweils N = 4, d.h. Mittelwerte ± unabhängigen Versuchsreihen, die in Dreifach-Parallel-Versuchen durchgeführt wurden).

Eine detaillierte Methodenbeschreibung kann der Literatur entnommen werden (M.Ch. Frink, H.-H. Hennies, W. Engelberger, M. Haurand und B. Wilffert (1996) Arzneim.-Forsch./Drug Res. 46 (III), 1029-1036).

Für die erfindungsgemäße Verbindung 2-[7,9-Dibrom-4-(4-methoxyphenyl)-6-methyl-3,4-dihydro-2H-pyrido[1,2-a]pyrimidin-2-yl]-cyclopropancarbonsäureethylester gemäß Beispiel 89 wurde eine deutliche Hemmung der Noradrenalinwiederaufnahme (10 µmol/l) von 53% gemessen. Dieses Ergebnis sowie die Ergebnisse für weitere erfindungsgemäße Verbindungen sind in der nachfolgenden Tabelle aufgeführt.

| **Verbindung gemäß Beispiel Nr.** | **NA-Uptakehemmung (10 µmol/l)** |
|---|---|
| 51 | 33 % |
| 56 | 35 % |
| 77 | 30 % |
| 78 | 43% |
| 84 | 33% |
| 89 | 53 % |

### Pharmazeutische Formulierung eines erfindungsgemäßen Arzneimittels

1 g des Hydrochlorids von 4-(3,4-Dimethoxyphenyl)-7-nitro-3.4-dihydro-2H-pyrido[1,2-a]pyrimidin-2-carbonsäure wurde in 1 l Wasser für Injektionszwecke bei Raumtemperatur gelöst und anschließend durch Zugabe von Natriumchlorid auf isotone Bedingungen eingestellt.

## Patentansprüche

1. Substituierte 3,4-Dihydro-pyrido[1,2-a]pyrimidine der allgemeinen Struktur I 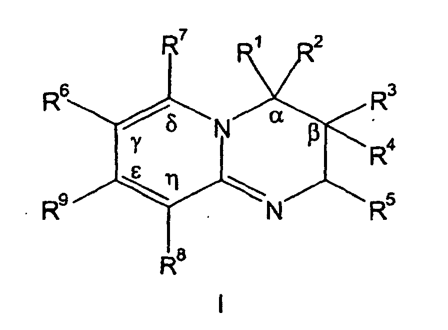 worin
R¹ und R² unabhängig voneinander H oder Phenyl oder Naphthyl, wobei Phenyl oder Naphthyl unsubstituiert oder ein- oder mehrfach mit OH oder OCH₃ substituiert sind, bedeuten, wobei mindestens ein Rest von R¹ und R² H ist,
R³ und R⁴ unabhängig voneinander H oder C₁₋₁₂-Alkyl, wobei C₁₋₁₂-Alkyl geradkettig oder verzweigt ist, bedeuten, wobei mindestens ein Rest von R³ und R⁴ H ist,
R⁵ C₃₋₈-Cycloalkyl, wobei Cycloalkyl gesättigt ist und unsubstituiert oder mit CO₂Ethyl substituiert ist, Furanyl substituiert mit NO₂, C(=O)R¹¹ oder CO₂R¹² bedeutet,
R⁶ und R⁸ beide Cl oder Br bedeuten,
R⁷ H oder C₁₋₁₂-Alkyl, wobei Alkyl geradkettig oder verzweigt ist, bedeutet,
R⁹ H bedeutet,
R¹¹ Phenyl ist,
R¹² C₁₋₈-Alkyl, wobei Alkyl geradkettig oder verzweigt ist, bedeutet,
sowie ihre pharmazeutisch annehmbaren Salze.

2. Verbindungen der allgemeinen Struktur I sowie ihre pharmazeutisch annehmbaren Salze nach Anspruch 1 in Form ihrer Racemate, in Form der reinen Enantiomeren oder in Form von Mischungen der Enantiomeren oder Diastereomeren in einem beliebigen Mischungsverhältnis.

3. Verbindungen der allgemeinen Struktur I nach einem der Ansprüche 1 oder 2 sowie ihre pharmazeutisch annehmbaren Salze, wobei einer der Reste R¹ und R² Naphthyl bedeutet oder Phenyl ein- oder mehrfach substituiert mit OH oder OCH₃, einer der Reste R³ und R⁴ H oder C₁₋₆-Alkyl bedeutet, und die beiden anderen Reste von R¹, R², R³ und R⁴ H bedeuten, R⁵ C₃₋₆-Cycloalkyl, Furanyl substituiert mit NO₂, C(=O)R¹¹ oder CO₂R¹² bedeutet, R⁶ und R⁸ beide Cl oder Br bedeuten, R⁷ H oder C₁₋₆-Alkyl bedeutet, R⁹ H bedeutet, R¹¹ Phenyl und R¹² C₁₋₆-Alkyl bedeuten,

4. Verbindungen der allgemeinen Struktur I nach Anspruch 3 sowie ihre pharmazeutisch annehmbaren Salze, wobei einer der Reste R¹ und R² 4-Hydroxyphenyl, 4-Methoxyphenyl, 4-Hydroxy-3-methoxyphenyl oder 2-Naphthyl bedeutet, einer der Reste R³ und R⁴ H oder Methyl bedeutet, und die beiden anderen Reste von R¹, R², R³ und R⁴ H bedeuten, R⁵ Cyclopropyl, 2-(C(=O)O-Ethyl)-cyclopropyl, Cyclohexyl, 5-Nitro-furan-2-yl, C(=O)Phenyl oder CO₂Ethyl bedeutet, R⁶ und R⁸ beide Cl oder Br bedeuten, R⁷ H oder Methyl bedeutet und R⁹ H bedeutet.

5. Verbindungen der allgemeinen Struktur I nach einem der Ansprüche 1 bis 4 sowie ihre pharmazeutisch annehmbaren Salze, wobei die Verbindungen ausgewählt sind aus:
[7,9-Dichlor-4-(4-hydroxy-3-methoxy-phenyl)-3-methyl-3,4-dihydro-2H-pyrido[1,2-a]pyrimidin-2-yl]-phenyl-methanon,
[7,9-Dibrom-4-(4-hydroxy-phenyl)-3,6-dimethyl-3,4-dihydro-2H-pyrido[1,2-a]pyrimidin-2-yl]-phenyl-methanon,
7,9-Dichlor-2-cyclohexyl-4-(4-methoxy-phenyl)-3,4-dihydro-2H-pyrido[1,2-a]pyrimidin,
7,9-Dibrom-2-cyclohexyl-4-(4-methoxy-phenyl)-3,4-dihydro-2H-pyrido[1,2-a]pyrimidin,
7,9-Dibrom-4-(3,4-dimethoxy-phenyl)-6-methyl-2-(5-nitro-furan-2-yl)-3,4-dihydro-2H-pyrido[1,2-a]pyrimidin,
2-[7,9-Dibrom-4-(4-methoxy-phenyl)-6-methyl-3,4-dihydro-2H-pyrido[1,2-a]pyrimidin-2-yl]-cyclopropancarbonsäureethylester.

6. Verfahren zur Herstellung von substituierten 3,4-Dihydro-pyrido[1,2-a]pyrimidinen der allgemeinen Struktur I sowie ihrer pharmazeutisch annehmbaren Salze, 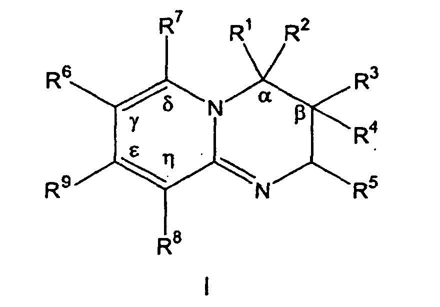 worin
R¹ und R² unabhängig voneinander H oder Phenyl oder Naphthyl, wobei Phenyl oder Naphtyl unsubstituiert oder ein- oder mehrfach mit OH und/oder OCH₃ substituiert sind, bedeuten, wobei mindestens ein Rest von R¹ und R² H ist,
R³ und R⁴ unabhängig voneinander H oder C₁₋₁₂-Alkyl, wobei C₁₋₁₂-Alkyl geradkettig oder verzweigt ist, wobei mindestens ein Rest von R³ und R⁴ H ist,
R⁵ C₃₋₈-Cycloalkyl, wobei Cycloalkyl gesättigt ist und unsubstituiert oder mit CO₂Ethyl substituiert ist, Furanyl substituiert mit NO₂, C(=O)R¹¹ oder CO₂R¹² bedeutet,
R⁶ und R⁸ beide Cl oder Br bedeuten,
R⁷ H oder C₁₋₁₂-Alkyl, wobei Alkyl geradkettig oder verzweigt ist, bedeutet,
R⁹ H bedeutet,
R¹¹ Phenyl ist,
R¹² C₁₋₈-Alkyl, wobei Alkyl geradkettig oder verzweigt ist, bedeutet,
**dadurch gekennzeichnet, daß**
ein Heteroarylamin der allgemeinen Struktur II 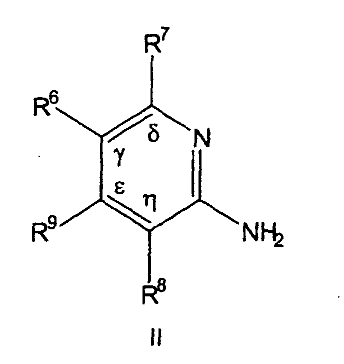 worin R⁶ , R⁷ , R⁸ und R⁹ wie oben definiert sind, mit einem Aldehyd der allgemeinen Struktur III 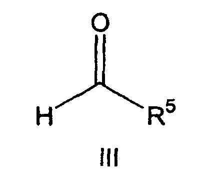 worin R⁵ wie oben definiert ist,
und einem Olefin der allgemeinen Struktur IV 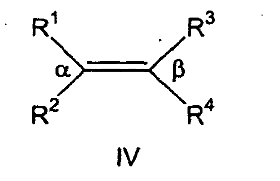 worin R¹, R², R³ und R⁴ wie oben definiert sind, in Gegenwart einer Säure umgesetzt wird.

7. Verfahren nach Anspruch 6, **dadurch gekennzeichnet, daß** die Umsetzung des Heteroarylamins der allgemeinen Struktur II mit dem Aldehyd der allgemeinen Struktur III und dem Olefin der allgemeinen Struktur IV in einem Eintopf-Verfahren durchgeführt wird.

8. Verfahren nach einem der Ansprüche 6 oder 7, **dadurch gekennzeichnet, daß** die Säure Trifluoressigsäure ist.

9. Verfahren nach einem der Ansprüche 6 bis 8, **dadurch gekennzeichnet, daß** die Umsetzung in einem organischen Lösungsmittel bei einer Temperatur von 0 bis 100 °C und einer Reaktionszeit von 0,25 bis 12 h durchgeführt wird.

10. Verfahren nach einem der Ansprüche 6 bis 9, **dadurch gekennzeichnet, daß** die Reaktion bei einer Temperatur von 15 bis 40 °C durchgeführt wird.

11. Verfahren nach einem der Ansprüche 6 bis 10, **dadurch gekennzeichnet, daß** das organische Lösungsmittel Acetonitril ist.

12. Arzneimittel, enthaltend mindestens eine Verbindung der allgemeinen Struktur I oder eines ihrer pharmazeutisch annehmbaren Salze 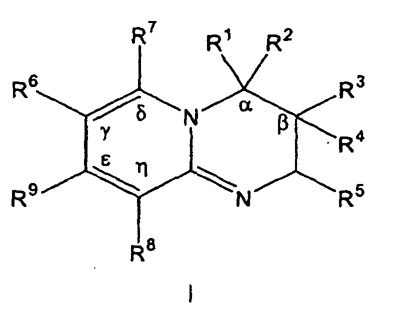 worin
R¹ und R² unabhängig voneinander H oder Phenyl oder Naphthyl, wobei Phenyl oder Naphthyl unsubstituiert oder ein- oder mehrfach mit OH oder OCH₃ substituiert sind, bedeuten, wobei ein Rest von R¹ und R² H ist,
R³ und R⁴ unabhängig voneinander H oder C₁₋₁₂-Alkyl, wobei C₁₋₁₂-Alkyl geradkettig oder verzweigt ist, bedeuten, wobei mindestens ein Rest von R³ und R⁴ H ist,
R⁵ C₃₋₈-Cycloalkyl, wobei Cycloalkyl gesättigt ist und unsubstituiert oder mit CO₂Ethyl substituiert ist, Furanyl substituiert mit NO₂, C(=O)R¹¹ oder CO₂R¹² bedeutet,
R⁶ und R⁸ beide Cl oder Br bedeuten,
R⁷ H oder C₁₋₁₂-Alkyl, wobei Alkyl geradkettig oder verzweigt ist, bedeutet,
R⁹ H bedeutet,
R¹¹ Phenyl bedeutet und
R¹² C₁₋₈-Alkyl, wobei Alkyl geradkettig oder verzweigt ist,

13. Verwendung einer Verbindung der allgemeinen Struktur I oder eines ihrer pharmazeutisch annehmbaren Salze 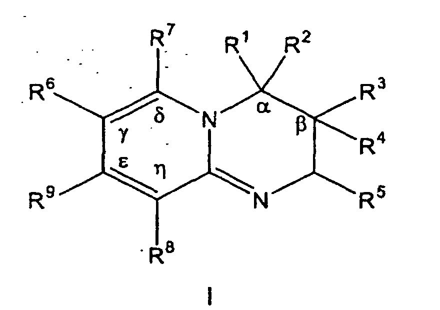 worin
R¹ und R² unabhängig voneinander H oder Phenyl oder Naphthyl, wobei Phenyl oder Naphthyl unsubstituiert oder ein- oder mehrfach mit OH oder OCH₃ substituiert sind, bedeuten, wobei mindestens ein Rest von R¹ und R² H ist,
R³ und R⁴ unabhängig voneinander H oder C₁₋₁₂-Alkyl, wobei C₁₋₁₂-Alkyl geradkettig oder verzweigt ist, bedeuten, wobei mindestens ein Rest von R³ und R⁴ H ist,
R⁵ C₃₋₈-Cycloalkyl, wobei Cycloalkyl gesättigt ist und unsubstituiert oder mit CO₂Ethyl substituiert ist, Furanyl substituiert mit NO₂, C(=O)R¹¹ oder CO₂R¹² bedeutet,
R⁶ und R⁸ beide Cl oder Br bedeuten,
R⁷ H oder C₁₋₁₂-Alkyl, wobei Alkyl geradkettig oder verzweigt ist, bedeutet,
R⁹ H bedeutet,
R¹¹ Phenyl ist,
R¹² C₁₋₈-Alkyl, wobei Alkyl geradkettig oder verzweigt ist, bedeutet,
zur Herstellung eines Arzneimittels zur Behandlung von Schmerz.

14. Verwendung einer Verbindung der allgemeinen Struktur I oder eines ihrer pharmazeutisch annehmbaren Salze 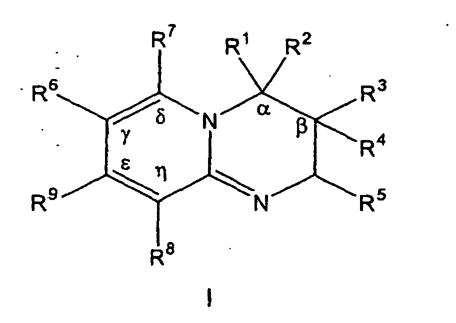 worin
R¹ und R² unabhängig voneinander H oder Phenyl oder Naphthyl, wobei Phenyl oder Naphthyl unsubstituiert oder ein- oder mehrfach mi OH oder OCH₃ substituiert sind, bedeuten, wobei mindestens ein Rest von R¹ und R² H ist,
R³ und R⁴ unabhängig voneinander H oder C₁₋₁₂-Alkyl, wobei C₁₋₁₂-Alkyl geradkettig oder verzweigt ist, bedeuten, wobei mindestens ein Rest von R³ und R⁴ H ist,
R⁵ C₃₋₈-Cycloalkyl, wobei Cycloalkyl gesättigt ist und unsubstituiert oder mit CO₂Ethyl substituiert ist, Furanyl substituiert mit NO₂, C(=O)R¹¹ oder CO₂R¹² bedeutet,
R⁶ und R⁸ beide Cl oder Br bedeuten,
R⁷ H oder C₁₋₁₂-Alkyl, wobei Alkyl geradkettig oder verzweigt ist, bedeutet,
R⁹ H bedeutet,
R¹¹ Phenyl ist,
R¹² C₁₋₈-Alkyl, wobei Alkyl geradkettig oder verzweigt ist, bedeutet,
zur Herstellung eines Arzneimittels zur Behandlung von Haminkontinenz, Juckreiz, Tinnitus aurium und/oder Diarrhoe.

15. Pharmazeutische Zusammensetzung, die mindestens eine Verbindung der allgemeinen Struktur I oder eines ihrer pharmazeutisch annehmbaren Salze 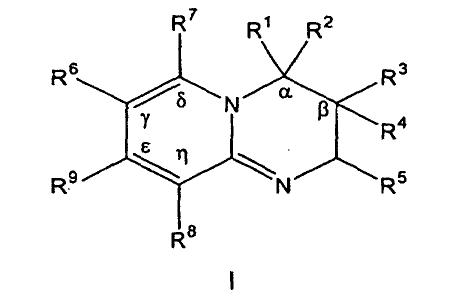 worin
R¹ und R² unabhängig voneinander H oder Phenyl oder Naphthyl, wobei Phenyl oder Naphthyl unsubstituiert oder ein- oder mehrfach mit OH oder OCH₃ substituiert sind, bedeuten, wobei mindestens ein Rest von R¹ und R² H ist,
R³ und R⁴ unabhängig voneinander H oder C₁₋₁₂-Alkyl, wobei C₁₋₁₂-Alkyl geradkettig oder verzweigt ist, bedeuten, wobei mindestens ein Rest von R³ und R⁴ H ist,
R⁵ C₃₋₈-Cycloalkyl, wobei Cycloalkyl gesättigt ist und unsubstituiert oder mit CO₂Ethyl substituiert ist, Furanyl substituiert mit NO₂, C(=O)R¹¹ oder CO₂R¹² bedeutet,
R⁶ und R⁸ beide Cl oder Br bedeuten,
R⁷ H oder C₁₋₁₂-Alkyl, wobei Alkyl geradkettig oder verzweigt ist, bedeutet,
R⁹ H bedeutet,
R¹¹ Phenyl ist,
R¹² C₁₋₈-Alkyl, wobei Alkyl geradkettig oder verzweigt ist, bedeutet,
sowie mindestens einen pharmazeutischen Hilfsstoff enthält.

## Claims

1. Substituted 3,4-dihydropyrido[1,2-a]pyrimidines of the general structure I 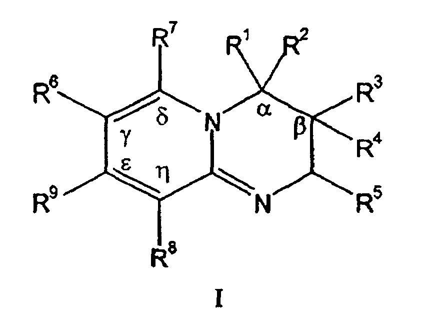 in which
R¹ and R² mutually independently mean H or phenyl or naphthyl, wherein phenyl or naphthyl are unsubstituted or mono- or polysubstituted with OH or OCH₃, wherein at least one residue of R¹ and R² is H,
R³ and R⁴ mutually independently mean H or C₁₋₁₂ alkyl, wherein C₁₋₁₂ alkyl is linear or branched, wherein at least one residue of R³ and R⁴ is H,
R⁵ means C₃₋₈ cycloalkyl, wherein cycloalkyl is saturated and is unsubstituted or substituted with CO₂ethyl, or means furanyl substituted with NO₂, C(=O)R¹¹ or CO₂R¹²,
R⁶ and R⁸ both mean Cl or Br,
R⁷ means H or C₁₋₁₂ alkyl, wherein alkyl is linear or branched,
R⁹ means H,
R¹¹ is phenyl,
R¹² means C₁₋₈ alkyl, wherein alkyl is linear or branched,
and the pharmaceutically acceptable salts thereof.

2. Compounds of the general structure I and the pharmaceutically acceptable salts thereof according to claim 1 in the form of the racemates thereof, in the form of the pure enantiomers or in the form of mixtures of the enantiomers or diastereomers in any desired mixing ratio.

3. Compounds of the general structure I according to one of claims 1 or 2 and the pharmaceutically acceptable salts thereof, wherein one of the residues R¹ and R² means naphthyl or means phenyl mono- or polysubstituted with OH or OCH₃, one of the residues R³ and R⁴ means H or C₁₋₆ alkyl, and the other two residues of R¹, R², R³ and R⁴ mean H, R⁵ means C₃₋₆ cycloalkyl, furanyl substituted with NO₂, C(=O)R¹¹ or CO₂R¹², R⁶ and R⁸ both mean Cl or Br, R⁷ means H or C₁₋₆ alkyl, R⁹ means H, R¹¹ means phenyl and R¹² means C₁₋₆ alkyl.

4. Compounds of the general structure I according to claim 3 and the pharmaceutically acceptable salts thereof, wherein one of the residues R¹ and R² means 4-hydroxyphenyl, 4-methoxyphenyl, 4-hydroxy-3-methoxyphenyl or 2-naphthyl, one of the residues R³ and R⁴ means H or methyl, and the other two residues of R¹, R², R³ and R⁴ mean H, R⁵ means cyclopropyl, 2-(C(=O)O-ethyl)-cyclopropyl, cyclohexyl, 5-nitro-2-furanyl, C(=O)phenyl or CO₂ethyl, R⁶ and R⁸ both mean Cl or Br, R⁷ means H or methyl and R⁹ means H.

5. Compounds of the general structure I according to one of claims 1 to 4 and the pharmaceutically acceptable salts thereof, wherein the compounds are selected from among:
[7,9-dichloro-4-(4-hydroxy-3-methoxyphenyl)-3-methyl-3,4-dihydro-2H-pyrido[1,2-a]-2-pyrimidinyl]-phenylmethanone,
[7,9-dibromo-4-(4-hydroxyphenyl)-3,6-dimethyl-3,4-dihydro-2H-pyrido[1,2-a]-2-pyrimidinyl]-phenylmethanone,
7,9-dichloro-2-cyclohexyl-4-(4-methoxyphenyl)-3,4-dihydro-2H-pyrido[1,2-a]pyrimidine,
7,9-dibromo-2-cyclohexyl-4-(4-methoxyphenyl)-3,4-dihydro-2H-pyrido[1,2-a]pyrimidine,
7,9-dibromo-4-(3,4-dimethoxyphenyl)-6-methyl-2-(5-nitro-2-furanyl)-3,4-dihydro-2H-pyrido[1,2-a]pyrimidine,
2-[7,9-dibromo-4-(4-methoxyphenyl)-6-methyl-3,4-dihydro-2H-pyrido[1,2-a]-2-pyrimidinyl]-cyclopropane carboxylic acid ethyl ester.

6. A process for the production of substituted 3,4-dihydropyrido[1,2-a]pyrimidines of the general structure I and the pharmaceutically acceptable salts thereof, 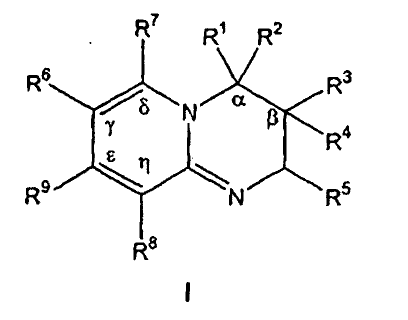 in which
R¹ and R² mutually independently mean H or phenyl or naphthyl, wherein phenyl or naphthyl are unsubstituted or mono- or polysubstituted with OH or OCH₃, wherein at least one residue of R¹ and R² is H,
R³ and R⁴ mutually independently mean H or C₁₋₁₂ alkyl, wherein C₁₋₁₂ alkyl is linear or branched, wherein at least one residue of R³ and R⁴ is H,
R⁵ means C₃₋₈ cycloalkyl, wherein cycloalkyl is saturated and is unsubstituted or substituted with CO₂ethyl, or means furanyl substituted with NO₂, C(=O)R¹¹ or CO₂R¹²,
R⁶ and R⁸ both mean Cl or Br,
R⁷ means H or C₁₋₁₂ alkyl, wherein alkyl is linear or branched,
R⁹ means H,
R¹¹ is phenyl,
R¹² means C₁₋₈ alkyl, wherein alkyl is linear or branched,
**characterised in that**
a heteroarylamine of the general structure II 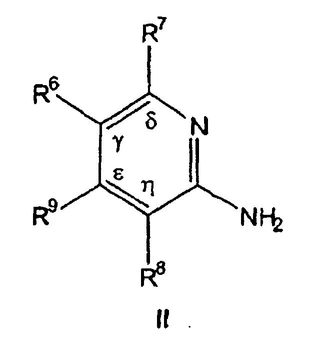 in which R⁶, R⁷, R⁸ and R⁹ are defined as above, is reacted in the presence of an acid with an aldehyde of the general structure III 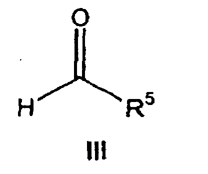 in which R⁵ is defined as above,
and an olefin of the general structure IV 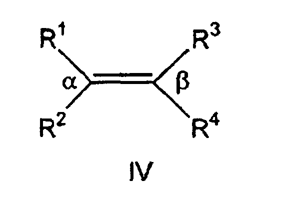 in which R¹, R², R³ and R⁴ are defined as above.

7. A process according to claim 6, **characterised in that** the reaction of the heteroarylamine of the general structure II with the aldehyde of the general structure III and the olefin of the general structure IV is performed in a single-vessel reaction.

8. A process according to either of claims 6 or 7, **characterised in that** the acid is trifluoroacetic acid.

9. A process according to one of claims 6 to 8, **characterised in that** the reaction is performed in an organic solvent at a temperature of 0 to 100°C and a reaction time of 0.25 to 12 h.

10. A process according to one of claims 6 to 9, **characterised in that** the reaction is performed at a temperature of 15 to 40°C.

11. A process according to one of claims 6 to 10, **characterised in that** the organic solvent is acetonitrile.

12. A pharmaceutical preparation containing at least one compound of the general structure I or a pharmaceutically acceptable salt thereof 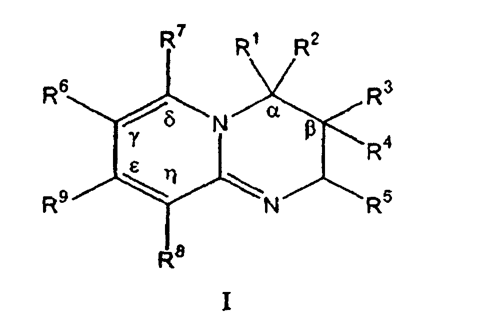 in which
R¹ and R² mutually independently mean H or phenyl or naphthyl, wherein phenyl or naphthyl are unsubstituted or mono- or polysubstituted with OH or OCH₃, wherein a residue of R¹ and R² is H,
R³ and R⁴ mutually independently mean H or C₁₋₁₂ alkyl, wherein C₁₋₁₂ alkyl is linear or branched, wherein at least one residue of R³ and R⁴ is H,
R⁵ means C₃₋₈ cycloalkyl, wherein cycloalkyl is saturated and is unsubstituted or substituted with CO₂ethyl, or means furanyl substituted with NO₂, C(=O)R¹¹ or CO₂R¹²,
R⁶ and R⁸ both mean Cl or Br,
R⁷ means H or C₁₋₁₂ alkyl, wherein alkyl is linear or branched,
R⁹ means H,
R¹¹ means phenyl and
R¹² means C₁₋₈ alkyl, wherein alkyl is linear or branched.

13. Use of a compound of the general structure I or a pharmaceutically acceptable salt thereof 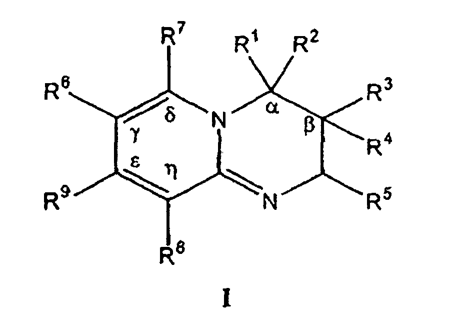 in which
R¹ and R² mutually independently mean H or phenyl or naphthyl, wherein phenyl or naphthyl are unsubstituted or mono- or polysubstituted with OH or OCH₃, wherein at least one residue of R¹ and R² is H,
R³ and R⁴ mutually independently mean H or C₁₋₁₂ alkyl, wherein C₁₋₁₂ alkyl is linear or branched, wherein at least one residue of R³ and R⁴ is H,
R⁵ means C₃₋₈ cycloalkyl, wherein cycloalkyl is saturated and is unsubstituted or substituted with CO₂ethyl, or means furanyl substituted with NO₂, C(=O)R¹¹ or CO₂R¹²,
R⁶ and R⁸ both mean Cl or Br,
R⁷ means H or C₁₋₁₂ alkyl, wherein alkyl is linear or branched,
R⁹ means H,
R¹¹ is phenyl,
R¹² means C₁₋₈ alkyl, wherein alkyl is linear or branched,
for the production of a pharmaceutical preparation for the treatment of pain.

14. Use of a compound of the general structure I or a pharmaceutically acceptable salt thereof 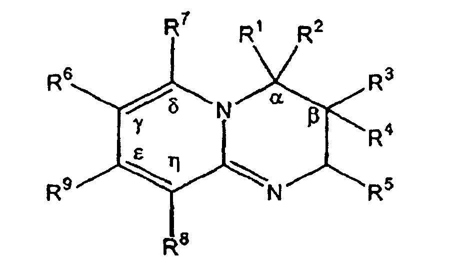 in which
R¹ and R² mutually independently mean H or phenyl or naphthyl, wherein phenyl or naphthyl are unsubstituted or mono- or polysubstituted with OH or OCH₃, wherein at least one residue of R¹ and R² is H,
R³ and R⁴ mutually independently mean H or C₁₋₁₂ alkyl, wherein C₁₋₁₂ alkyl is linear or branched, wherein at least one residue of R³ and R⁴ is H,
R⁵ means C₃₋₈ cycloalkyl, wherein cycloalkyl is saturated and is unsubstituted or substituted with CO₂ethyl, or means furanyl substituted with NO₂, C(=O)R¹¹ or CO₂R¹²,
R⁶ and R⁸ both mean Cl or Br,
R⁷ means H or C₁₋₁₂ alkyl, wherein alkyl is linear or branched,
R⁹ means H,
R¹¹ is phenyl,
R¹² means C₁₋₈ alkyl, wherein alkyl is linear or branched,
for the production of a pharmaceutical preparation for the treatment of urinary incontinence, pruritus, tinnitus and/or diarrhoea.

15. A pharmaceutical composition, which contains at least one compound of the general structure I or a pharmaceutically acceptable salt thereof 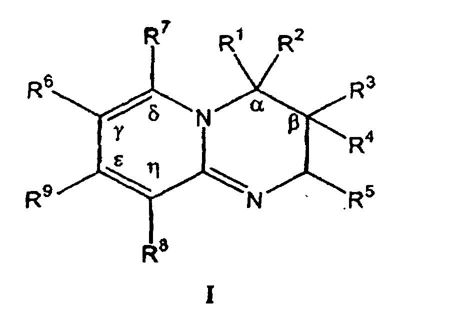 in which
R¹ and R² mutually independently mean H or phenyl or naphthyl, wherein phenyl or naphthyl are unsubstituted or mono- or polysubstituted with OH or OCH₃, wherein at least one residue of R¹ and R² is H,
R³ and R⁴ mutually independently mean H or C₁₋₁₂ alkyl, wherein C₁₋₁₂ alkyl is linear or branched, wherein at least one residue of R³ and R⁴ is H,
R⁵ means C₃₋₈ cycloalkyl, wherein cycloalkyl is saturated and is unsubstituted or substituted with CO₂ethyl, or means furanyl substituted with NO₂, C(=O)R¹¹ or CO₂R¹²,
R⁶ and R⁸ both mean Cl or Br,
R⁷ means H or C₁₋₁₂ alkyl, wherein alkyl is linear or branched,
R⁹ means H,
R¹¹ is phenyl,
R¹² means C₁₋₈ alkyl, wherein alkyl is linear or branched,
together with at least one pharmaceutical auxiliary substance.

## Revendications

1. 3,4-dihydro-pyrido[1,2-a]pyrimidine substituée de formule générale I 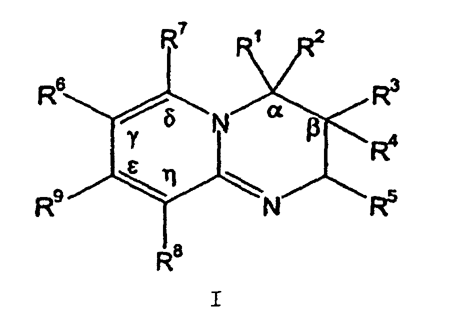 dans laquelle
R¹ et R² représentent, indépendamment l'un de l'autre, H ou un reste phényle ou naphtyle, le reste phényle ou naphtyle étant non substitué ou substitué une ou plusieurs fois avec un radical OH ou OCH₃, au moins l'un des restes R¹ et R² représentant H,
R³ et R⁴ représentent, indépendamment l'un de l'autre, H ou un reste alkyle en C₁ à C₁₂, le reste alkyle en C₁ à C₁₂ étant linéaire ou ramifié, au moins l'un des restes R³ et R⁴ représentant H,
R⁵ est un reste cycloalkyle en C₃ à C₈, le reste cycloalkyle étant saturé et non substitué ou substitué avec un radical CO₂éthyle, un reste furannyle substitué avec un radical NO₂, C(=O)R¹¹ ou CO₂R¹²,
R⁶ et R⁸ représentent tous deux Cl ou Br,
R⁷ représente H ou un reste alkyle en C₁ à C₁₂, ce reste alkyle étant linéaire ou ramifié,
R⁹ représente H,
R¹¹ est un reste phényle,
R¹² est un reste alkyle en C₁ à C₈, ce reste alkyle étant linéaire ou ramifié,
ainsi que leurs sels acceptables du point de vue pharmaceutique.

2. Composés de formule générale I et leurs sels acceptables du point de vue pharmaceutique suivant la revendication 1, sous forme de leurs racémates, sous forme des énantiomères purs ou sous forme de mélanges des énantiomères ou des diastéréoisomères dans un rapport de mélange quelconque.

3. Composés de formule générale I suivant l'une des revendications 1 et 2 ainsi que leurs sels acceptables du point de vue pharmaceutique, où l'un des restes R¹ et R² désigne un reste naphtyle ou représente un reste phényle substitué une ou plusieurs fois avec un radical OH ou OCH₃, l'un des restes R³ et R⁴ désigne H ou un reste alkyle en C₁ à C₆, et les deux autres restes de R¹, R², R³ et R⁴ représentent H, R⁵ est un reste cycloalkyle en C₃ à C₆, furannyle substitué avec un radical NO₂, C(=O)R¹¹ ou CO₂R¹², R⁶ et R⁸ représentent tous deux Cl ou Br, R⁷ représente H ou un reste alkyle en C₁ à C₆, R⁹ représente H, R¹¹ est un reste phényle et R¹² est un reste alkyle en C₁ à C₆.

4. Composés de formule générale I suivant la revendication 3 ainsi que leurs sels acceptables du point de vue pharmaceutique, où l'un des restes R¹ et R² est un reste 4-hydroxyphényle, 4-méthoxyphényle, 4-hydroxy-3-méthoxyphényle ou 2-naphtyle, l'un des restes R³ et R⁴ représente H ou un reste méthyle, et les deux autres restes de R¹, R², R³ et R⁴ représentent H, R⁵ est un reste cyclopropyle, 2-(C(=O)-éthyl)cyclopropyle, cyclohexyle, 5-nitro-furanne-2-yle, C(=O)phényle ou CO₂éthyle, R⁶ et R⁸ représentent tous deux Cl ou Br, R⁷ représente H ou le reste méthyle et R⁹ représente H.

5. Composés de formule générale I suivant l'une des revendications 1 à 4 ainsi que leurs sels acceptables du point de vue pharmaceutique, les composés étant choisis entre :
la [7,9-dichloro-4-(4-hydroxy-3-méthoxyphényl)-3-méthyl-3,4-dihydro-2H-pyrido[1,2-a]pyrimidine-2-yl]-phénylméthanone,
la [7,9-dibromo-4-(4-hydroxyphényl)-3,6-diméthyl-3,4-dihydro-2H-pyrido[1,2-a]pyrimidine-2-yl]-phénylméthanone,
la 7,9-dichloro-2-cyclohexyl-4-(4-méthoxyphényl)-3,4-dihydro-2H-pyrido[1,2-a]pyrimidine,
la 7,9-dibromo-2-cyclohexyl-4-(4-méthoxyphényl)-3,4-dihydro-2H-pyrido[1,2-a]pyrimidine,
la 7,9-dibromo-4-(3,4-diméthoxyphényl)-6-méthyl-2-(5-nitro-furanne-2-yl)-3,4-dihydro-2H-pyrido[1,2-a]pyrimidine,
l'ester éthylique de l'acide 2-[7,9-dibromo-4-(4-méthoxy-phényl)-6-méthyl-3,4-dihydro-2H-pyrido[1,2-a]pyrimidine-2-yl]-cyclopropanecarboxylique.

6. Procédé de production de 3,4-dihydro-pyrido[1,2-a]pyrimidines substituées de formule générale I ainsi que de leurs sels acceptables du point de vue pharmaceutique 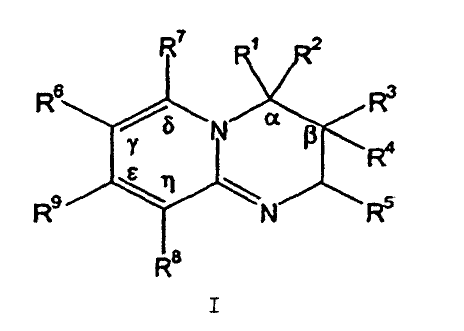 dans laquelle
R¹ et R² représentent, indépendamment l'un de l'autre, H ou un reste phényle ou naphtyle, le reste phényle ou naphtyle étant non substitué ou substitué une ou plusieurs fois avec un radical OH ou OCH₃, au moins l'un des restes R¹ et R² représentant H,
R³ et R⁴ représentent, indépendamment l'un de l'autre, H ou un reste alkyle en C₁ à C₁₂, le reste alkyle en C₁ à C₁₂ étant linéaire ou ramifié, au moins l'un des restes R³ et R⁴ représentant H,
R⁵ est un reste cycloalkyle en C₃ à C₈, le reste cycloalkyle étant saturé et non substitué ou substitué avec un radical CO₂éthyle, un reste furannyle substitué avec un radical NO₂, C(=O)R¹¹ ou CO₂R¹²,
R⁶ et R⁸ représentent tous deux Cl ou Br,
R⁷ représente H ou un reste alkyle en C₁ à C₁₂, ce reste alkyle étant linéaire ou ramifié,
R⁹ représente H,
R¹¹ est un reste phényle,
R¹² est un reste alkyle en C₁ à C₈, ce reste alkyle étant linéaire ou ramifié,
**caractérisé en ce qu'**une hétéroarylamine de formule générale II 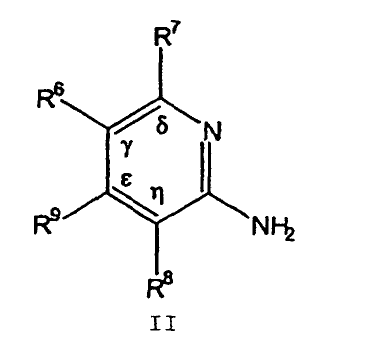 dans laquelle R⁶, R⁷, R⁸ et R⁹ sont tels que définis ci-dessus,
est amenée à réagir avec un aldéhyde de formule générale III 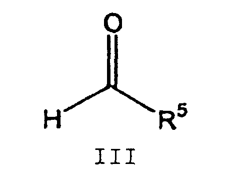 dans laquelle R⁵ est tel que défini ci-dessus,
et avec une oléfine de formule générale IV 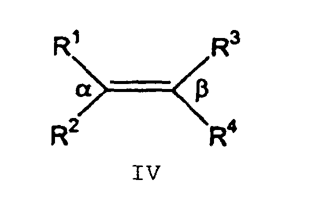 dans laquelle R¹, R², R³ et R⁴ sont tels que définis ci-dessus, en présence d'un acide.

7. Procédé suivant la revendication 6, **caractérisé en ce que** la réaction de l'hétéroarylamine de formule générale II avec l'aldéhyde de formule générale III et l'oléfine de formule générale IV est conduite dans un procédé en récipient unique.

8. Procédé suivant l'une des revendications 6 et 7, **caractérisé en ce que** l'acide est l'acide trifluoracétique.

9. Procédé suivant l'une des revendications 6 à 8, **caractérisé en ce que** la réaction est conduite dans un solvant organique à une température de 0 à 100 °C et pendant une durée de réaction de 0,25 à 12 h.

10. Procédé suivant l'une des revendications 6 à 9, **caractérisé en ce que** la réaction est conduite à une température de 15 à 40 °C.

11. Procédé suivant l'une des revendications 6 à 10, **caractérisé en ce que** le solvant organique est l'acétonitrile.

12. Médicament contenant au moins un composé de formule générale I ou l'un de ses sels acceptables du point de vue pharmaceutique 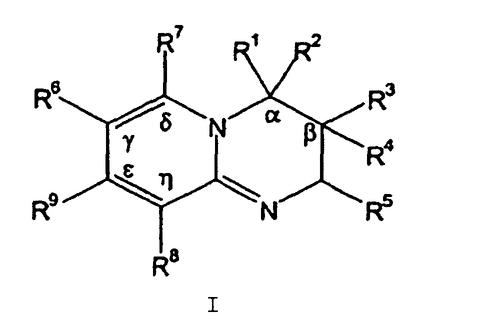 dans laquelle
R¹ et R² représentent, indépendamment l'un de l'autre, H ou un reste phényle ou naphtyle, le reste phényle ou naphtyle étant non substitué ou substitué une ou plusieurs fois avec un radical OH ou OCH₃, au moins l'un des restes R¹ et R² représentant H,
R³ et R⁴ représentent, indépendamment l'un de l'autre, H ou un reste alkyle en C₁ à C₁₂, le reste alkyle en C₁ à C₁₂ étant linéaire ou ramifié, au moins l'un des restes R³ et R⁴ représentant H,
R⁵ est un reste cycloalkyle en C₃ à C₈, le reste cycloalkyle étant saturé et non substitué ou substitué avec un radical CO₂éthyle, un reste furannyle substitué avec un radical NO₂, C(=O)R¹¹ ou CO₂R¹²,
R⁶ et R⁸ représentent tous deux Cl ou Br,
R⁷ représente H ou un reste alkyle en C₁ à C₁₂, ce reste alkyle étant linéaire ou ramifié,
R⁹ représente H,
R¹¹ est un reste phényle, et
R¹² est un reste alkyle en C₁ à C₈, ce reste alkyle étant linéaire ou ramifié.

13. Utilisation d'un composé de formule générale I ou de l'un de ses sels acceptables du point de vue pharmaceutique 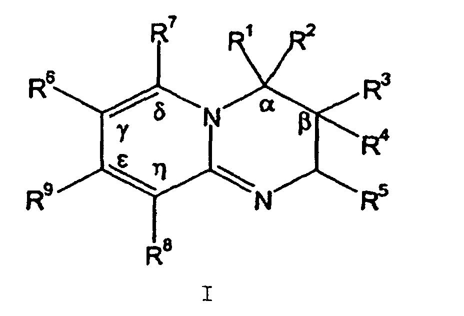 dans laquelle
R¹ et R² représentent, indépendamment l'un de l'autre, H ou un reste phényle ou naphtyle, le reste phényle ou naphtyle étant non substitué ou substitué une ou plusieurs fois avec un radical OH ou OCH₃, au moins l'un des restes R¹ et R² représentant H,
R³ et R⁴ représentent, indépendamment l'un de l'autre, H ou un reste alkyle en C₁ à C₁₂, le reste alkyle en C₁ à C₁₂ étant linéaire ou ramifié, au moins l'un des restes R³ et R⁴ représentant H,
R⁵ est un reste cycloalkyle en C₃ à C₈, le reste cycloalkyle étant saturé et non substitué ou substitué avec un radical CO₂éthyle, un reste furannyle substitué avec un radical NO₂, C(=O)R¹¹ ou CO₂R¹²,
R⁶ et R⁸ représentent tous deux Cl ou Br,
R⁷ représente H ou un reste alkyle en C₁ à C₁₂, ce reste alkyle étant linéaire ou ramifié,
R⁹ représente H,
R¹¹ est un reste phényle,
R¹² est un reste alkyle en C₁ à C₈, ce reste alkyle étant linéaire ou ramifié,
pour la préparation d'un médicament destiné au traitement de la douleur.

14. Utilisation d'un composé de formule générale I ou de l'un de ses sels acceptables du point de vue pharmaceutique 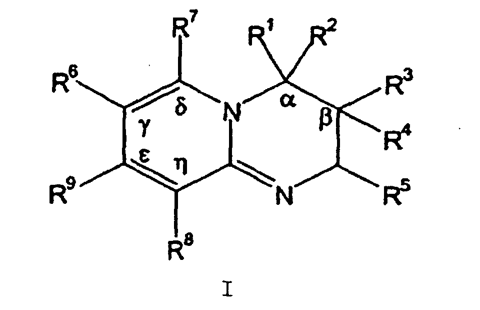 dans laquelle
R¹ et R² représentent, indépendamment l'un de l'autre, H ou un reste phényle ou naphtyle, le reste phényle ou naphtyle étant non substitué ou substitué une ou plusieurs fois avec un radical OH ou OCH₃, au moins l'un des restes R¹ et R² représentant H,
R³ et R⁴ représentent, indépendamment l'un de l'autre, H ou un reste alkyle en C₁ à C₁₂, le reste alkyle en C₁ à C₁₂ étant linéaire ou ramifié, au moins l'un des restes R³ et R⁴ représentant H,
R⁵ est un reste cycloalkyle en C₃ à C₈, le reste cycloalkyle étant saturé et non substitué ou substitué avec un radical CO₂éthyle, un reste furannyle substitué avec un radical NO₂, C(=O)R¹¹ ou CO₂R¹²,
R⁶ et R⁸ représentent tous deux Cl ou Br,
R⁷ représente H ou un reste alkyle en C₁ à C₁₂, ce reste alkyle étant linéaire ou ramifié,
R⁹ représente H,
R¹¹ est un reste phényle,
R¹² est un reste alkyle en C₁ à C₈, ce reste alkyle étant linéaire ou ramifié,
pour la préparation d'un médicament destiné au traitement de l'incontinence urinaire, du prurit, des acouphènes et/ou de la diarrhée.

15. Composition pharmaceutique, qui contient au moins un composé de formule générale I ou l'un de ses sels acceptables du point de vue pharmaceutique 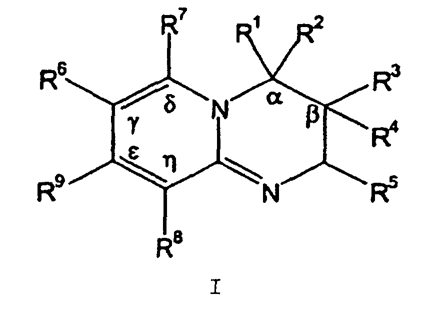 dans laquelle
R¹ et R² représentent, indépendamment l'un de l'autre, H ou un reste phényle ou naphtyle, le reste phényle ou naphtyle étant non substitué ou substitué une ou plusieurs fois avec un radical OH ou OCH₃, au moins l'un des restes R¹ et R² représentant H,
R³ et R⁴ représentent, indépendamment l'un de l'autre, H ou un reste alkyle en C₁ à C₁₂, le reste alkyle en C₁ à C₁₂ étant linéaire ou ramifié, au moins l'un des restes R³ et R⁴ représentant H,
R⁵ est un reste cycloalkyle en C₃ à C₈, le reste cycloalkyle étant saturé et non substitué ou substitué avec un radical CO₂éthyle, un reste furannyle substitué avec un radical NO₂, C(=O)R¹¹ ou CO₂R¹²,
R⁶ et R⁸ représentent tous deux Cl ou Br,
R⁷ représente H ou un reste alkyle en C₁ à C₁₂, ce reste alkyle étant linéaire ou ramifié,
R⁹ représente H,
R¹¹ est un reste phényle,
R¹² est un reste alkyle en C₁ à C₈, ce reste alkyle étant linéaire ou ramifié,
ainsi qu'au moins une substance auxiliaire pharmaceutique.
